# EUROPÄISCHE PATENTSCHRIFT

(11) **EP 2 506 966 B1**
(45) Veröffentlichungstag und Bekanntmachung des Hinweises auf die Patenterteilung: **16.08.2017**
(21) Anmeldenummer: 10784512.5
(22) Anmeldetag: 29.11.2010
(51) Int. Cl.: B01J 37/03, B01J 37/18, B01J 23/835, C07C 213/02, C07D 295/03, C07D 295/027

(54) **KATALYSATOR UND VERFAHREN ZUR HERSTELLUNG EINES AMINS**
CATALYST AND METHOD FOR PRODUCING AN AMINE
CATALYSEUR ET PROCÉDÉ DE PRODUCTION D'UNE AMINE

(30) Priorität: 03.12.2009 EP 09177914
(43) Veröffentlichungstag der Anmeldung: 10.10.2012
(73) Patentinhaber: BASF SE, 67056 Ludwigshafen am Rhein (DE)
(72) Erfinder: KUBANEK, Petr, 68163 Mannheim (DE); MÄGERLEIN, Wolfgang, 68165 Mannheim (DE); MELDER, Johann-Peter, 67459 Böhl-Iggelheim (DE); HEIDEMANN, Thomas, 68519 Viernheim (DE)
(74) Vertreter: BASF IP Association
(86) Internationale Anmeldenummer: PCT/EP2010/068375
(87) Internationale Veröffentlichungsnummer: WO 2011/067199

(56) Entgegenhaltungen:
- EP-A2- 0 839 575
- WO-A1-2008/006750

## Beschreibung

Die vorliegende Erfindung betrifft aluminiumoxid-, kupfer-, nickel- und kobalthaltige Katalysatoren und ein Verfahren zur Herstellung eines Amins durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, in Gegenwart eines geträgerten, kupfer-, nickel- und kobalthaltigen Katalysators.

Die Verfahrensprodukte finden u.a. Verwendung als Zwischenprodukte bei der Herstellung von Kraftstoffadditiven (US 3,275,554 A; DE 21 25 039 A und DE 36 11 230 A), Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren.

EP 963 975 A1 und EP 1 106 600 A2 (beide BASF AG) beschreiben Verfahren zur Herstellung von Aminen aus Alkoholen bzw. Aldehyden oder Ketonen und Stickstoffverbindungen unter Verwendung eines Katalysators, dessen katalytisch aktive Masse 22-40 Gew.-% (bzw. 22-45 Gew.-%) sauerstoffhaltige Verbindungen des Zirkoniums, 1-30 Gew.-% sauerstoffhaltige Verbindungen des Kupfers und jeweils 15-50 Gew.-% (bzw. 5-50 Gew.-%) sauerstoffhaltige Verbindungen des Nickels und Kobalts enthält. Auch WO 03/076386 A und EP 1 431 271 A1 (beide BASF AG) lehren Katalysatoren des o. g. Typs für Aminierungen. Ein Sn-Gehalt wird nicht gelehrt.

EP 514 692 A2 (BASF AG) betrifft ein Verfahren zur Herstellung von Aminen aus Alkanolen in Gegenwart von Katalysatoren enthaltend Cu, Ni, ggf. Co, ZrO₂ und/oder Al₂O₃. Der bevorzugte Katalysator besteht aus 55 Gew.-% Al₂O₃, 36 Gew.-% Cu und 7 Gew.-% Ni (Beispiel 1). Ein Sn-Gehalt wird nicht gelehrt.

WO 03/051508 A1 (Huntsman Petrochemical Corp.) betrifft Verfahren zur Aminierung von Alkoholen unter Verwendung von spezifischen Cu/Ni/Zr/Sn - haltigen Katalysatoren, die in einer weiteren Ausgestaltung Cr statt Zr enthalten (siehe Seite 4, Zeilen 10-16). Die in dieser WO-Anmeldung beschriebenen Katalysatoren enthalten kein Aluminiumoxid und kein Kobalt.

WO 2007/036496 A (BASF AG) beschreibt ein Verfahren zur Herstellung von Aminodiglykol (ADG) und Morpholin durch Umsetzung von Diethylenglykol (DEG) mit Ammoniak in Gegenwart eines Übergangsmetall-Heterogenkatalysators, wobei die katalytisch aktive Masse des Katalysators vor der Behandlung mit Wasserstoff sauerstoffhaltige Verbindungen des Aluminiums und/oder Zirkoniums, Kupfers, Nickels und Kobalts enthält und der Katalysatorformkörper spezifische Dimensionen aufweist. Ein Sn-Gehalt wird nicht gelehrt.

DE 28 44 984 A1 (Shell Int. Res.) beschreibt Verfahren zur Herstellung eines Amins durch Umsetzen eines Alkohols, Aldehyds oder Ketons mit bis zu 25 C-Atomen mit Ammoniak, einem primären oder sekundären Amin an einem Katalysator, der Cu, Sn und ggf. Alkali- oder Erdalkalimetall auf einem porösen Träger, wie zum Beispiel Aluminiumoxid, umfasst. Diese Katalysatoren enthalten kein Nickel und kein Kobalt.

EP 839 574 A2 und EP 839 575 A2 (beide BASF AG) beschreiben Katalysatoren zur Aminierung von Alkoholen, die Ni, Co, Cu, Ru auf einem porösem Metalloxid-Träger, wie z. B. Aluminiumoxid, Siliciumdioxid, Titandioxid, Zirkoniumdioxid u. a., umfassen. Unter zahlreichen möglichen Promotoren ist Sn erwähnt. Katalysatoraktivität und Katalysatorstabilität sind verbesserungswürdig.

US 6,147,261 (Shell Oil Corp.) lehrt Nickel- und/oder Kobalt-Katalysatoren zur Aminierung von bestimmten Hydroxyalkanalen, die optional ein Träger, wie z. B. Aluminiumoxid, Magnesiumoxid, Silica u. a., enthalten. Bevorzugte Katalysatoren sind Raney-Kobalt und Raney-Nickel. Die beschriebenen Katalysatoren enthalten kein Sn.

US 6,534,441 B1 (Union Carbide) beschreibt Katalysatoren für die reduktive Aminierung von niederen aliphatischen Alkanderivaten, deren Aktivmasse von einem synergistischen Effekt von Ni und Re profitierten soll. Solche Katalysatoren basieren auf einem Aluminosilikat-Träger mit 5-65 Gew.-% Silica. Die Katalysatoren können auch einen Promotor aus zahlreichen Gruppen des Periodensystems, darunter Gruppe IVA (Sn), IB (Cu), VIII (Ni, Co), enthalten.

WO 98/26868 A1 (Batelle Memorial Institute) beschreibt Katalysatoren für Reaktionen in der wässriger Phase auf Basis Ni, die ein Promotor aus der Gruppe Cu, Sn, Ag, Re, Ru oder deren Kombination enthalten. Der Gehalt an Promotor liegt bei < 5 Gew.-%. Die Aminierung von Alkoholen/Aldehyden/Ketonen wird nicht beschrieben. Ebenso gehört ein Aluminiumoxid-Träger nicht zu den beschriebenen Trägern.

WO 2004/084887 A1 (DuPont) beansprucht ein Verfahren zur Herstellung von Pyrrolidonderivaten aus Levulinsäure und aromatischen Aminen (reduktive Aminierung). Zahlreiche verschiedene Katalysatoren enthaltend insbesondere Edelmetalle auf verschiedenen Trägern, wie u. a. auch Alumina, werden verwendet. Sn ist nicht enthalten.

DE 19 53 263 A (BASF AG) offenbart Katalysatoren enthaltend Kobalt, Nickel und Kupfer auf Aluminiumoxid mit einem Metallgehalt von 5 bis 80 Gew.-%, insbesondere 10 bis 30 Gew.-%, bezogen auf den gesamten Katalysator, wobei die Katalysatoren, berechnet auf den Metallgehalt, 70 bis 95 Gew.-% einer Mischung aus Kobalt und Nickel und 5 bis 30 Gew.-% Kupfer enthalten. Beispielsweise besitzt der Katalysator die Zusammensetzung 10 Gew.-% CoO, 10 Gew.-% NiO und 4 Gew.-% CuO auf Al₂O₃. Der Katalysator enthält kein Sn und Katalysatoraktivität und Katalysatorstabilität sind verbesserungswürdig.

WO 2008/006750 A1 (BASF AG) betrifft bestimmte Pb, Bi, Sn, Sb und/oder In - dotierte, zirkoniumdioxid-, kupfer-, nickel- und kobalthaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt.

WO 2009/080507 A1 (BASF SE) betrifft bestimmte Sn- und Co-dotierte, zirkoniumdioxid-, kupfer- und nickelhaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt.

WO 2009/080506 A1 (BASF SE) beschreibt bestimmte Pb, Bi, Sn, Mo, Sb und/oder P - dotierte, zirkoniumdioxid-, nickel- und eisenhaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt. Bevorzugt enthalten die Katalysatoren kein Cu und kein Co.

WO 2009/080508 A1 (BASF SE) lehrt bestimmte Pb, Bi, Sn und/oder Sb - dotierte, zirkoniumdioxid-, kupfer-, nickel-, kobalt und eisenhaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt.

WO 2009/114438 A2 (Huntsman Petrochem. Corp.) betrifft die Aminierung von Cyclohexandimethanol in Gegenwart von Wasserstoff und ZrO₂-geträgerten Metallkatalysatoren, z. B. ZrO₂/Cu/Ni/Sn.

Eine parallele europäische Patentanmeldung mit gleichem Anmeldetag (BASF SE) betrifft bestimmte dotierte aluminiumoxid-, kupfer-, nickel-, kobalt- und zinnhaltige Katalysatoren und ihre Verwendung in Verfahren zur Herstellung eines Amins aus einem primären oder sekundären Alkohol, Aldehyd und/oder Keton.

Beim Einsatz der sehr aktiven Katalysatoren des Stands der Technik, insbesondere auch der Katalysatoren gemäß EP 963 975 A1 und EP 1 106 600 A2 (s. o.), kann es bei den Edukten (Alkohole, Aldehyde, Ketone) bei erhöhter Temperatur verstärkt zur Decarbonylierung der (gegebenenfalls intermediär entstandenen) Carbonylfunktion kommen. Die Bildung von Methan durch Hydrierung von Kohlenmonoxid (CO) führt aufgrund der großen freiwerdenden Hydrierwärme zu einer 'Durchgehgefahr', d. h. einem unkontrollierten Temperaturanstieg im Reaktor. Wird CO durch Amine abgefangen, kommt es zur Bildung von Methylgruppen-haltigen Nebenkomponenten.

Darüber hinaus kann es beim Einsatz der sehr aktiven Aminierungskatalysatoren aus der Stand der Technik, vor allem denen auf Basis von Zirkoniumdioxid, zu unerwünschter Etherspaltung kommen und die Ausbeute an wirtschaftlich interessanten Produkten wie z. B. ADG und Morpholin (MOR) wird damit verbesserungswürdig.

Bei der Aminierung von Diethylenglykol (DEG) kommt es beispielsweise verstärkt zur Bildung von unerwünschtem Methoxyethanol bzw. Methoxyethylamin. Das Methoxyethanol ist giftig, kann aufgrund seiner physikalischen Eigenschaften aus Morpholin nur schlecht abgetrennt werden und kann damit zu Problemen hinsichtlich Spezifikation und Produktqualität führen.

Beim Beispiel der Aminierung von Diethylenglykol (DEG) wird die "Decarbonylierung" insbesondere als die Summe von unerwünschten Komponenten (Methanol, Methoxyethanol, Methoxyethylamin, N-Methylmorpholin und Methoxy-Ethyl-Morpholin) betrachtet, die gemäß dem Reaktionsnetzwerk aus DEG über Methoxyethanol entstehen:

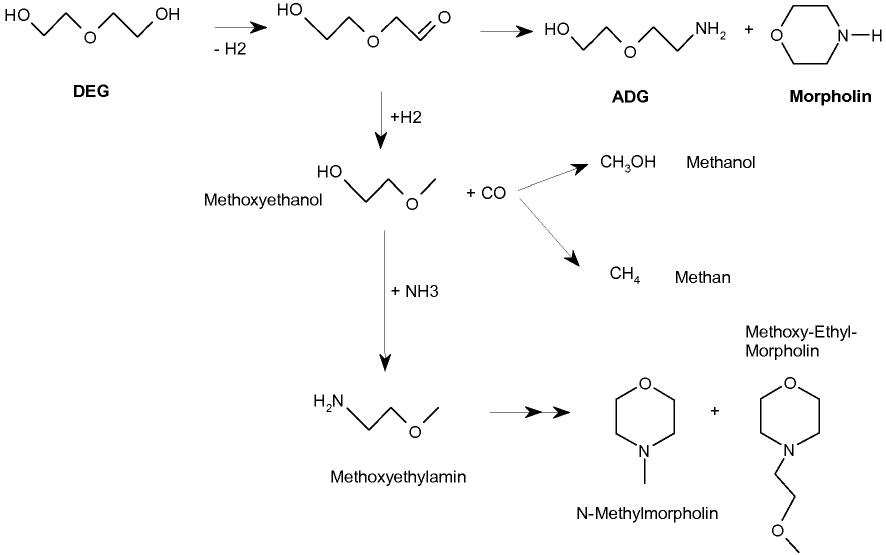

Als Reaktionsmechanismus der Aminierung von primären oder sekundären Alkoholen wird angenommen, dass der Alkohol zunächst an einem Metallzentrum zum entsprechenden Aldehyd dehydriert wird. Hierbei kommt dem Kupfer oder auch Nickel als Dehydrierkomponente vermutlich besondere Bedeutung zu. Werden Aldehyde zur Aminierung eingesetzt, entfällt dieser Schritt.

Der gebildete bzw. eingesetzte Aldehyd kann durch Reaktion mit Ammoniak oder primärem oder sekundärem Amin unter Wasserabspaltung und anschließender Hydrierung aminiert werden. Diese Kondensation des Aldehyds mit der o. g. Stickstoffverbindung wird vermutlich durch saure Zentren des Katalysators katalysiert. In einer unerwünschten Nebenreaktion kann der Aldehyd aber auch decarbonyliert werden, d. h. dass die Aldehydfunktion als CO abgespalten wird. Die Decarbonylierung bzw. Methanisierung findet vermutlich an einem metallischen Zentrum statt. Das CO wird an dem Hydrierkatalysator zu Methan hydriert, so dass die Methanbildung das Ausmaß der Decarbonylierung anzeigt. Durch die Decarbonylierung entstehen die oben erwähnten unerwünschten Nebenprodukte wie z. B. im o. g. Fall Methoxyethanol und/oder Methoxyethylamin.

Es handelt sich bei der erwünschten Kondensation des Aldehyds mit Ammoniak oder primärem oder sekundärem Amin und bei der unerwünschten Decarbonylierung des Aldehyds um Parallelreaktionen, von denen die erwünschte Kondensation vermutlich säurekatalysiert ist, während die unerwünschte Decarbonylierung durch metallische Zentren katalysiert ist.

Der vorliegenden Erfindung lag die Aufgabe zugrunde, die Wirtschaftlichkeit bisheriger Verfahren zur hydrierenden Aminierung von Aldehyden oder Ketonen und der Aminierung von Alkoholen zu verbessern und einem Nachteil oder mehreren Nachteilen des Stands der Technik, insbesondere den o. g. Nachteilen, abzuhelfen. Es sollten Katalysatoren gefunden werden, die technisch in einfacher Weise herzustellen sind und die es erlauben, die o. g. Aminierungen mit hohem Umsatz, hoher Ausbeute, Raum-Zeit-Ausbeuten (RZA), Selektivität bei gleichzeitig hoher mechanischer Stabilität des Katalysatorformkörpers und geringer 'Durchgehgefahr', durchzuführen. Die Katalysatoren sollen demnach eine hohe Aktivität und unter den Reaktionsbedingungen eine hohe chemische und mechanische Stabilität aufweisen. Darüber hinaus sollte der Einsatz der Katalysatoren in entsprechenden Aminierungsverfahren, in denen aufgrund der chemischen Struktur der Edukte lineare und zyklische Verfahrensprodukte resultieren können, mit verbesserter Selektivität zu dem/den linearen Verfahrensprodukt/en führen. Insbesondere sollten auch Katalysatoren gefunden werden, die zu höheren Ausbeuten an wirtschaftlich interessanten Produkten wie z. B. Aminodiglykol und Morpholin ausgehend von DEG führen.

[Raum-Zeit-Ausbeuten werden angegeben in ,Produktmenge / (Katalysatorvolumen ● Zeit)' (kg/(l_{Kat.} ● h)) und/oder,Produktmenge / (Reaktorvolumen ● Zeit)' (kg/(l_{Reaktor} ● h)]. Demgemäß wurde ein Verfahren zur Herstellung eines Amins durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, in Gegenwart eines geträgerten, kupfer-, nickel- und kobalthaltigen Katalysators gefunden, welches dadurch gekennzeichnet ist, dass die katalytisch aktive Masse des Katalysators vor dessen Reduktion mit Wasserstoff sauerstoffhaltige Verbindungen des Aluminiums, Kupfers, Nickels und Kobalts und im Bereich von 0,2 bis 5,0 Gew.-% sauerstoffhaltige Verbindungen des Zinns, berechnet als SnO, enthält.

Darüber hinaus wurden Katalysatoren gefunden, deren katalytisch aktive Masse vor deren Reduktion mit Wasserstoff sauerstoffhaltige Verbindungen des Aluminiums, Kupfers, Nickels und Kobalts und im Bereich von 0,2 bis 5,0 Gew.-% sauerstoffhaltige Verbindungen des Zinns, berechnet als SnO, enthalten.

Insbesondere wurden Katalysatoren, deren katalytisch aktive Masse vor deren Reduktion mit Wasserstoff im Bereich von
15 bis 80 Gew.-% sauerstoffhaltige Verbindungen des Aluminiums, berechnet als Al₂O₃,
1 bis 20 Gew.-% sauerstoffhaltige Verbindungen des Kupfers, berechnet als CuO,
5 bis 35 Gew.-% sauerstoffhaltige Verbindungen des Nickels, berechnet als NiO,
5 bis 35 Gew.-% sauerstoffhaltige Verbindungen des Kobalts , berechnet als CoO, und 0,2 bis 5,0 Gew.-% sauerstoffhaltige Verbindungen des Zinns, berechnet als SnO, enthält,
und ihre Verwendung im o. g. Aminierungsverfahren, insbesondere im Verfahren zur Umsetzung von DEG mit Ammoniak, gefunden.

Alle Angaben zur Zusammensetzung der katalytisch aktiven Masse der erfindungsgemäßen und im erfindungsgemäßen Verfahren verwendeten Katalysatoren beziehen sich auf die katalytisch aktive Masse vor deren Reduktion mit Wasserstoff.

In der Literatur findet sich kein Hinweis, dass die spezifische Kombination von Nickel, Kupfer, Kobalt, Zinn und Aluminiumoxid insbesondere zu einem synergistischen Effekt führt und diese Katalysatoren vorteilhaft sind im Vergleich zum Stand der Technik bei der Aminierung von Alkoholen/Aldehyden/Ketonen, insbesondere besser hinsichtlich Gesamtselektivität und Verfahrenssicherheit sind.

Erfindungsgemäß wurde erkannt, dass die Aktivität des Katalysators zur Aminierung von primären oder sekundären Alkoholen, Aldehyden und/oder Ketonen in Gegenwart von H₂, z. B. die Aminierung von Diethylenglykol (DEG) mit Ammoniak zu Aminodiglykol und Morpholin, durch den Gehalt der Aluminiumoxid-Kupfer-Nickel-Katalysatoren an Kobalt und den zusätzlichen spezifischen Gehalt an Sn, im wesentlichen zumindest gleich bleibt, gleichzeitig aber das Ausmaß der unerwünschten Decarbonylierungsreaktion abnimmt und damit die Selektivität der Aminierungsreaktion zunimmt. Gleichzeitig wird das Ausmaß der unerwünschten Hochsiederbildung unterdrückt und damit wird die Selektivität der Aminierungsreaktion verbessert.

Das Verfahren kann kontinuierlich oder diskontinuierlich durchgeführt werden. Bevorzugt ist eine kontinuierliche Fahrweise.

Für die Synthese in der Gasphase werden die Edukte gezielt, bevorzugt in einem Kreisgasstrom, verdampft und gasförmig dem Reaktor zugeführt. Geeignete Amine für eine Gasphasensynthese sind Amine, die aufgrund ihrer Siedepunkte und der Siedepunkte ihrer Edukte verfahrenstechnisch im Rahmen der Prozessparameter in der Gasphase gehalten werden können. Das Kreisgas dient zum einen der Verdampfung der Edukte und zum anderen als Reaktionspartner für die Aminierung.

In der Kreisgasfahrweise werden die Ausgangsstoffe (Alkohol, Aldehyd und/oder Keton, Wasserstoff und die Stickstoffverbindung) in einem Kreisgasstrom verdampft und gasförmig dem Reaktor zugeführt.

Die Edukte (Alkohol, Aldehyd und/oder Keton, die Stickstoffverbindung) können auch als wässrige Lösungen verdampft und mit dem Kreisgasstrom auf das Katalysatorbett geleitet werden.

Bevorzugte Reaktoren sind Rohrreaktoren. Beispiele für geeignete Reaktoren mit Kreisgasstrom finden sich in Ullmann's Encyclopedia of Industrial Chemistry, 5th Ed., Vol. B 4, Seiten 199-238, "Fixed-Bed Reactors".

Alternativ erfolgt die Umsetzung vorteilhaft in einem Rohrbündelreaktor oder in einer Monostranganlage.

Bei einer Monostranganlage kann der Rohrreaktor, in dem die Umsetzung erfolgt, aus einer Hintereinanderschaltung mehrerer (z. B. zweier oder dreier) einzelner Rohrreaktoren bestehen. Optional ist hier vorteilhaft eine Zwischeneinspeisung von Feed (enthaltend das Edukt und/oder Ammoniak und/oder H₂) und/oder Kreisgas und/oder Reaktoraustrag aus einem nachgeschalteten Reaktor möglich.

Die Kreisgasmenge liegt bevorzugt im Bereich von 40 bis 1500 m³ (bei Betriebsdruck) / [m³ Katalysator (Schüttvolumen) • h], insbesondere im Bereich von 100 bis 700 m³ (bei Betriebsdruck) / [m³ Katalysator (Schüttvolumen) • h].

Das Kreisgas enthält bevorzugt mindestens 10, besonders 50 bis 100, ganz besonders 80 bis 100, Vol.% H₂.

Für die Synthese in der Flüssigphase sind alle Edukte und Produkte geeignet, welche schwer verdampfbar oder thermisch labil sind. In diesen Fällen kommt als weiterer Vorteil hinzu, dass das auf eine Verdampfung und Rekondensation des Amins im Prozess verzichtet werden kann.

Im erfindungsgemäßen Verfahren werden die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten.
In diesem Zusammenhang wird das oxidische Trägermaterial Aluminiumoxid (Al₂O₃) als zur katalytisch aktiven Masse gehörig gewertet.

Die Katalysatoren werden dergestalt eingesetzt, dass man die katalytisch aktive, zu Pulver vermahlene Masse in das Reaktionsgefäß einbringt oder, dass man die katalytisch aktive Masse nach Mahlung, Vermischung mit Formhilfsmitteln, Formung und Temperung als Katalysatorformkörper - beispielsweise als Tabletten, Kugeln, Ringe, Extrudate (z. B. Stränge) - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und der o. g. Katalysatorträgermaterialien definiert und enthält im wesentlichen die folgenden Bestandteile:
Aluminiumoxid (Al₂O₃), sauerstoffhaltige Verbindungen des Kupfers, Nickels und Kobalts und sauerstoffhaltige Verbindungen des Zinns.

Die Summe der o. g. Bestandteile der katalytisch aktiven Masse beträgt üblicherweise 70 bis 100 Gew.-%, bevorzugt 80 bis 100 Gew.-%, besonders bevorzugt 90 bis 100 Gew.-%, besonders > 95 Gew.-%, ganz besonders > 98 Gew.-%, insbesondere > 99 Gew.-%, z. B. besonders bevorzugt 100 Gew.-%.

Die katalytisch aktive Masse der erfindungsgemäßen und im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems, enthalten.

Beispiele für solche Elemente bzw. deren Verbindungen sind:
Übergangsmetalle, wie Mn bzw. MnO₂ W bzw. Wolframoxide, Ta bzw. Tantaloxide, Nb bzw. Nioboxide oder Nioboxalat, V bzw. Vanadiumoxide bzw. Vanadylpyrophosphat; Lanthanide, wie Ce bzw. CeO₂ oder Pr bzw. Pr₂O₃; Erdalkalimetalloxide, wie SrO; Erdalkalimetallcarbonate, wie MgCO₃, CaCO₃ und BaCO₃; Boroxid (B₂O₃).

Bevorzugt enthält die katalytisch aktive Masse der erfindungsgemäßen und im erfindungsgemäßen Verfahren eingesetzten Katalysatoren kein Rhenium, kein Ruthenium, kein Eisen und/oder kein Zink, jeweils weder in metallischer (Oxidationsstufe = 0) noch in einer ionischen (Oxidationsstufe ≠ 0), insbesondere oxidierten, Form.

Bevorzugt enthält die katalytisch aktive Masse der erfindungsgemäßen und im erfindungsgemäßen Verfahren eingesetzten Katalysatoren kein Silber und/oder Molybdän, jeweils weder in metallischer (Oxidationsstufe = 0) noch in einer ionischen (Oxidationsstufe ≠ 0), insbesondere oxidierten, Form.

In einer besonders bevorzugten Ausführungsform enthält die katalytisch aktive Masse der erfindungsgemäßen und im erfindungsgemäßen Verfahren eingesetzten Katalysatoren keine weitere katalytisch aktive Komponente, weder in elementarer (Oxidationsstufe = 0) noch in ionischer (Oxidationsstufe ≠ 0) Form.
In der besonders bevorzugten Ausführungsform ist die katalytisch aktive Masse nicht mit weiteren Metallen oder Metallverbindungen dotiert.
Bevorzugt sind jedoch aus der Metallgewinnung von Cu, Co, Ni, Sn herrührende übliche Begleit-Spurenelemente hiervon ausgenommen.

Bevorzugt enthält die katalytisch aktive Masse des Katalysators keine sauerstoffhaltigen Verbindungen des Siliziums, Zirkoniums und/oder des Chroms.

Die katalytisch aktive Masse des Katalysators enthält vor dessen Reduktion mit Wasserstoff im Bereich von 0,2 bis 5,0 Gew.-%, besonders im Bereich von 0,4 bis 4,0 Gew.-%, weiter besonders im Bereich von 0,6 bis 3,0 Gew.-%, weiter besonders bevorzugt im Bereich von 0,7 bis 2,5 Gew.-%, sauerstoffhaltige Verbindungen des Zinns, berechnet als SnO.

Die katalytisch aktive Masse des Katalysators enthält vor dessen Reduktion mit Wasserstoff bevorzugt im Bereich von 5,0 bis 35 Gew.-%, besonders im Bereich von 10 bis 30 Gew.-%, weiter besonders im Bereich von 12 bis 28 Gew.-%, ganz besonders 15 bis 25 Gew.-%, sauerstoffhaltige Verbindungen des Kobalts, berechnet als CoO.

Die katalytisch aktive Masse des Katalysators enthält vor dessen Reduktion mit Wasserstoff weiterhin bevorzugt im Bereich von
15 bis 80 Gew.-%, besonders 30 bis 70 Gew.-%, weiter besonders 35 bis 65 Gew.-%, sauerstoffhaltige Verbindungen des Aluminiums, berechnet als Al₂O₃,
1 bis 20 Gew.-%, besonders 2 bis 18 Gew.-%, weiter besonders 5 bis 15 Gew.-%, sauerstoffhaltige Verbindungen des Kupfers, berechnet als CuO, und
5 bis 35 Gew.-%, besonders 10 bis 30 Gew.-%, weiter besonders 12 bis 28 Gew.-%, ganz besonders 15 bis 25 Gew.-%, sauerstoffhaltige Verbindungen des Nickels, berechnet als NiO.

Das Molverhältnis von Nickel zu Kupfer beträgt bevorzugt größer 1, besonders bevorzugt größer 1,2, weiter besonders bevorzugt im Bereich von 1,8 bis 8,5.

Die BET-Oberfläche (ISO 9277:1995) der erfindungsgemäßen und im erfindungsgemäßen Verfahren eingesetzten Katalysatoren liegt bevorzugt im Bereich von 30 bis 250 m²/g, besonders im Bereich von 90 bis 200 m²/g, weiter besonders im Bereich von 130 bis 190 m²/g. Diese Bereiche werden insbesondere durch Calciniertemperaturen bei der Katalysatorherstellung im Bereich von 400 bis 600 °C, besonders 420 bis 550 °C, (vgl. unten) erzielt.

Zur Herstellung der im erfindungsgemäßen Verfahren verwendeten Katalysatoren sind verschiedene Verfahren möglich. Sie sind beispielsweise durch Peptisieren pulvriger Mischungen der Hydroxide, Carbonate, Oxide und/oder anderer Salze der Komponenten mit Wasser und nachfolgendes Extrudieren und Tempern (Wärmebehandlung) der so erhaltenen Masse erhältlich.

Bevorzugt werden zur Herstellung der erfindungsgemäßen Katalysatoren Fällungsmethoden angewandt. So können sie beispielsweise durch eine gemeinsame Fällung der Nickel-, Kobalt-, Kupfer- und Sn-Komponenten aus einer diese Elemente enthaltenden, wässrigen Salzlösung mittels Basen in Gegenwart einer Aufschlämmung einer schwerlöslichen, sauerstoffhaltigen Aluminiumverbindung und anschließendes Waschen, Trocknen und Calcinieren des erhaltenen Präzipitats erhalten werden. Als schwerlösliche, sauerstoffhaltige Aluminiumverbindungen können beispielsweise Aluminiumoxid, Aluminiumoxidhydrat, Aluminiumphosphate, -borate und -silikate Verwendung finden. Die Aufschlämmungen der schwerlöslichen Aluminiumverbindungen können durch Suspendieren feinkörniger Pulver dieser Verbindungen in Wasser unter kräftigem Rühren hergestellt werden. Vorteilhaft werden diese Aufschlämmungen durch Ausfällen der schwerlöslichen Aluminiumverbindungen aus wässrigen Aluminiumsalzlösungen mittels Basen erhalten.

Bevorzugt werden die erfindungsgemäßen Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Base, - beispielsweise Natriumcarbonat, Natriumhydroxid, Kaliumcarbonat oder Kaliumhydroxid - versetzt, bis die Fällung vollständig ist. Es kann auch mit Alkalimetall-freien Basen wie Ammoniak, Ammoniumcarbonat, Ammoniumhydrogencarbonat, Ammoniumcarbamat, Ammoniumoxalat, Ammoniummalonat, Urotropin, Harnstoff, etc. gearbeitet werden. Die Art der verwendeten Salze ist im Allgemeinen nicht kritisch: Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im Allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d. h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden zu den erfindungsgemäßen Katalysatoren wie üblich weiterverarbeitet. Zunächst werden die Niederschläge gewaschen. Über die Dauer des Waschvorgangs und über die Temperatur und Menge des Waschwassers kann der Gehalt an Alkalimetall, das durch die als Fällungsmittel eventuell verwendete (Mineral)base zugeführt wurde, beeinflusst werden. Im Allgemeinen wird durch Verlängerung der Waschzeit oder Erhöhung der Temperatur des Waschwassers der Gehalt an Alkalimetall abnehmen. Nach dem Waschen wird das Fällgut im Allgemeinen bei 80 bis 200 °C, vorzugsweise bei 100 bis 150 °C, getrocknet und danach calciniert. Die Calcinierung wird im Allgemeinen bei Temperaturen zwischen 300 und 800 °C, vorzugsweise bei 400 bis 600 °C, insbesondere bei 420 bis 550 °C ausgeführt.

Die erfindungsgemäßen Katalysatoren können auch durch Tränkung von Aluminiumoxid (Al₂O₃), das beispielsweise in Form von Pulver oder Formkörpern, wie Strängen, Tabletten, Kugeln oder Ringen, vorliegt, hergestellt werden.

Das Aluminiumoxid wird beispielsweise in der amorphen, gamma-, theta- und/oder delta- Form, als Aluminiumoxohydroxid (Böhmit), bevorzugt in der amorphen Form eingesetzt.

Die Herstellung von Formkörpern kann nach den üblichen Verfahren erfolgen.

Die Tränkung erfolgt ebenfalls nach den üblichen Verfahren, wie z. B. in A. B. Stiles, Catalyst Manufacture - Laboratory and Commercial Preparations, Marcel Dekker, New York (1983) beschrieben, durch Aufbringung einer jeweils entsprechenden Metallsalzlösung in einer oder mehreren Tränkstufen, wobei als Metallsalze z. B. entsprechende Nitrate, Acetate oder Chloride verwendet werden. Die Masse wird im Anschluss an die Tränkung getrocknet und optional calziniert.

Die Tränkung kann nach der sogenannten "incipient wetness"-Methode erfolgen, bei der das Aluminiumoxid entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und optional zu calzinieren. Die mehrstufige Tränkung ist vorteilhaft besonders dann anzuwenden, wenn das Aluminiumoxid mit einer größeren Metallmenge beaufschlagt werden soll.

Zur Aufbringung der Metallkomponenten auf das Aluminiumoxid kann die Tränkung gleichzeitig mit allen Metallsalzen oder in beliebiger Reihenfolge der einzelnen Metallsalze nacheinander erfolgen.

Anschließend werden die durch Tränkung hergestellten Katalysatoren getrocknet und bevorzugt auch calciniert, z. B. bei den bereits oben angegebenen Calciniertemperaturbereichen.

Nach der Calcinierung wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Presse zu Formlingen, z. B. Tabletten, verpresst und tempert. Die Tempertemperaturen entsprechen dabei bevorzugt den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d. h. insbesondere als Oxide und Mischoxide.

Die z. B. wie oben beschreiben hergestellten Katalysatoren werden als solche gelagert und ggf. gehandelt. Vor ihrem Einsatz als Katalysatoren werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden.

Zur Vorreduktion werden die Katalysatoren zunächst bei bevorzugt 150 bis 200 °C über einen Zeitraum von z. B. 12 bis 20 Stunden einer Stickstoff-Wasserstoff-Atmosphäre ausgesetzt und anschließend noch bis zu ca. 24 Stunden bei bevorzugt 200 bis 400 °C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Ein weiterer Vorteil der erfindungsgemäßen Katalysatoren ist deren mechanische Stabilität, d. h. deren Härte. Die mechanische Stabilität kann durch die Messung der sogenannten Seitendruckfestigkeit bestimmt werden. Hierzu wird der Katalysatorformkörper, z. B. die Katalysatortablette, zwischen zwei parallelen Platten mit zunehmender Kraft belastet, wobei diese Belastung z. B. auf der Mantelseite von Katalysatortabletten erfolgen kann, bis ein Bruch des Katalysatorformkörpers eintritt. Die beim Bruch des Katalysatorformkörpers registrierte Kraft ist die Seitendruckfestigkeit.

Das erfindungsgemäße Verfahren wird bevorzugt kontinuierlich durchgeführt, wobei der Katalysator bevorzugt als Festbett im Reaktor angeordnet ist. Dabei ist sowohl eine Anströmung des Katalysatorfestbetts von oben als auch von unten möglich. Der Gasstrom wird dabei durch Temperatur, Druck und Menge so eingestellt, dass auch schwerer siedende (hoch siedende) Reaktionsprodukte in der Gasphase verbleiben.

Das Aminierungsmittel kann bezüglich der zu aminierenden alkoholischen Hydroxylgruppe bzw. Aldehydgruppe bzw. Ketogruppe in stöchiometrischen, unter- oder überstöchiometrischen Mengen eingesetzt werden.

Bevorzugt wird im Falle der Aminierung von Alkoholen, Aldehyden oder Ketonen mit primären oder sekundären Aminen das Amin in ca. stöchiometrischer Menge oder geringfügig überstöchiometrischer Menge pro Mol zu aminierender alkoholischer Hydroxylgruppe, Aldehydgruppe oder Ketogruppe eingesetzt.

Die Aminkomponente (Stickstoffverbindung) wird bevorzugt in der 0,90- bis 100-fachen molaren Menge, insbesondere in der 1,0- bis 10-fachen molaren Menge, jeweils bezogen auf den/das eingesetzte/n Alkohol, Aldehyd und/oder Keton eingesetzt.

Speziell Ammoniak wird im Allgemeinen mit einem 1,5 bis 250-fachen, bevorzugt 2 bis 100-fachen, insbesondere 2 bis 10-fachen molaren Überschuss pro Mol umzusetzender alkoholischer Hydroxylgruppe, Aldehydgruppe oder Ketogruppe eingesetzt. Höhere Überschüsse sowohl an Ammoniak als auch an primären oder sekundären Aminen sind möglich.

Bevorzugt wird eine Abgasmenge von 5 bis 800 Normkubikmeter/h, insbesondere 20 bis 300 Normkubikmeter/h, gefahren. (Normkubikmeter = auf Normalbedingungen umgerechnetes Volumen).

Die Aminierung der primären oder sekundären Alkoholgruppen, Aldehydgruppen oder Ketogruppen des Edukts kann in der Flüssigphase oder in der Gasphase durchgeführt werden. Bevorzugt ist das Festbettverfahren in der Gasphase.

Beim Arbeiten in der Flüssigphase leitet man die Edukte (Alkohol, Aldehyd oder Keton plus Ammoniak oder Amin) simultan in flüssiger Phase bei Drücken von im Allgemeinen 5 bis 30 MPa (50-300 bar), bevorzugt 5 bis 25 MPa, besonders bevorzugt 15 bis 25 MPa, und Temperaturen von im Allgemeinen 80 bis 350 °C, besonders 100 bis 300 °C, bevorzugt 120 bis 270 °C, besonders bevorzugt 130 bis 250 °C, insbesondere 170 bis 230 °C, inklusive Wasserstoff über den Katalysator, der sich üblicherweise in einem bevorzugt von außen beheizten Festbettreaktor befindet. Es ist dabei sowohl eine Rieselfahrweise als auch eine Sumpffahrweise möglich. Die Katalysatorbelastung liegt im Allgemeinen im Bereich von 0,05 bis 5, bevorzugt 0,1 bis 2, besonders bevorzugt 0,2 bis 0,6, kg Alkohol, Aldehyd oder Keton pro Liter Katalysator (Schüttvolumen) und Stunde. Gegebenenfalls kann eine Verdünnung der Edukte mit einem geeigneten Lösungsmittel, wie Tetrahydrofuran, Dioxan, N-Methylpyrrolidon oder Ethylenglykoldimethylether, erfolgen. Es ist zweckmäßig, die Reaktanden bereits vor der Zuführung in das Reaktionsgefäß zu erwärmen, und zwar bevorzugt auf die Reaktionstemperatur.

Beim Arbeiten in der Gasphase werden die gasförmigen Edukte (Alkohol, Aldehyd oder Keton plus Ammoniak oder Amin) in einem zur Verdampfung ausreichend groß gewählten Gasstrom, bevorzugt Wasserstoff, bei Drücken von im Allgemeinen 0,1 bis 40 MPa (1 bis 400 bar), bevorzugt 0,1 bis 10 MPa, besonders bevorzugt 0,1 bis 5 MPa, in Gegenwart von Wasserstoff über den Katalysator geleitet. Die Temperaturen für die Aminierung von Alkoholen betragen im Allgemeinen 80 bis 350 °C, besonders 100 bis 300 °C, bevorzugt 120 bis 270 °C, besonders bevorzugt 160 bis 250 °C. Die Reaktionstemperaturen bei der hydrierenden Aminierung von Aldehyden und Ketonen betragen im Allgemeinen 80 bis 350 °C, besonders 90 bis 300 °C, bevorzugt 100 bis 250 °C. Es ist dabei sowohl eine Anströmung des Katalysatorfestbetts von oben als auch von unten möglich. Den erforderlichen Gasstrom erhält man bevorzugt durch eine Kreisgasfahrweise.
Die Katalysatorbelastung liegt im Allgemeinen im Bereich von 0,01 bis 2, bevorzugt 0,05 bis 0,5, kg Alkohol, Aldehyd oder Keton pro Liter Katalysator (Schüttvolumen) und Stunde.

Der Wasserstoff wird der Reaktion im Allgemeinen in einer Menge von 5 bis 400 I, bevorzugt in einer Menge von 50 bis 200 I pro Mol Alkohol-, Aldehyd- oder Ketonkomponente zugeführt, wobei die Literangaben jeweils auf Normalbedingungen umgerechnet wurden (S.T.P.).
Die Aminierung von Aldehyden bzw. Ketonen unterscheidet sich in der Durchführung von der Aminierung von Alkoholen dadurch, dass bei der Aminierung von Aldehyden und Ketonen mindestens stöchiometrische Mengen an Wasserstoff vorhanden sein müssen.

Sowohl beim Arbeiten in der Flüssigphase als auch beim Arbeiten in der Gasphase ist die Anwendung höherer Temperaturen und höherer Gesamtdrücke und Katalysatorbelastungen möglich. Der Druck im Reaktionsgefäß, welcher sich aus der Summe der Partialdrücke des Aminierungsmittels, des Alkohols, Aldehyds bzw. Ketons und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Sowohl beim kontinuierlichen Arbeiten in der Flüssigphase als auch beim kontinuierlichen Arbeiten in der Gasphase kann das überschüssige Aminierungsmittel zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Das im Zuge der Umsetzung gebildete Reaktionswasser (jeweils ein Mol pro Mol umgesetzte Alkoholgruppe, Aldehydgruppe bzw. Ketogruppe) wirkt sich im Allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Aus dem Reaktionsaustrag werden, nachdem dieser zweckmäßigerweise entspannt worden ist, der überschüssige Wasserstoff und das gegebenenfalls vorhandene überschüssige Aminierungsmittel entfernt und das erhaltene Reaktionsrohprodukt gereinigt, z. B. durch eine fraktionierende Rektifikation. Geeignete Aufarbeitungsverfahren sind z. B. in EP 1 312 600 A und EP 1 312 599 A (beide BASF AG) beschrieben. Das überschüssige Aminierungsmittel und der Wasserstoff werden vorteilhaft wieder in die Reaktionszone zurückgeführt. Das gleiche gilt für die eventuell nicht vollständig umgesetzte Alkohol-, Aldehyd- bzw. Ketonkomponente.

Unumgesetzte Edukte und gegebenenfalls anfallende geeignete Nebenprodukte können wieder in die Synthese zurückgeführt werden. Nicht umgesetzte Edukte können in diskontinuierlicher oder kontinuierlicher Fahrweise nach Kondensation der Produkte im Abscheider in dem Kreisgasstrom erneut über das Katalysatorbett geströmt werden.

Aminierungsmittel im erfindungsgemäßen Verfahren sind neben Ammoniak primäre und sekundäre Amine.

Mit dem erfindungsgemäßen Verfahren herstellbar sind z. B. Amine der Formel I 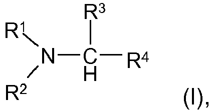 in der
- R¹, R²: Wasserstoff (H), Alkyl, wie C₁₋₂₀-Alkyl, Cycloalkyl, wie C₃₋₁₂-Cycloalkyl, Alkoxyalkyl, wie C₂₋₃₀-Alkoxyalkyl, Dialky-laminoalkyl, wie C₃₋₃₀-Dialkylaminoalkyl, Aryl, Aralkyl, wie C₇₋₂₀-Aralkyl, und Alkylaryl, wie C₇₋₂₀-Alkylaryl, oder gemeinsam -(CH₂)ⱼ-X-(CH₂)ₖ-,
- R³, R⁴: Wasserstoff (H), Alkyl, wie C₁₋₂₀-Alkyl, Cycloalkyl, wie C₃₋₁₂-Cycloalkyl, Hydroxyalkyl, wie C₁₋₂₀-Hydroxyalkyl, Aminoalkyl, wie C₁₋₂₀-Aminoalkyl, Hydroxyalkylaminoalkyl, wie C₂₋₂₀- Hydroxyalkylaminoalkyl, Alkoxyalkyl, wie C₂₋₃₀-Alkoxyalkyl, Dialkylaminoalkyl, wie C₃₋₃₀-Dialkylamino-alkyl, Alkylaminoalkyl, wie C₂₋₃₀-Alkylaminoalkyl, R⁵-(OCR⁶R⁷CR⁸R⁹)ₙ-(OCR⁶R⁷), Aryl, Heteroaryl, Aralkyl, wie C₇₋₂₀-Aralkyl, Heteroarylalkyl, wie C₄₋₂₀-Heteroarylalkyl, Alkylaryl, wie C₇₋₂₀-Alkylaryl, Alkylheteroaryl, wie C₄₋₂₀-Alkylheteroaryl, und Y-(CH₂)ₘ-NR⁵-(CH₂)_{q} oder gemeinsam -(CH₂)ₗ-X-(CH₂)ₘ- oder
- R² und R⁴: gemeinsam -(CH₂)ₗ-X-(CH₂)ₘ-,
- R⁵, R¹⁰: Wasserstoff (H), Alkyl, wie C₁₋₄-Alkyl, Alkylphenyl, wie C₇₋₄₀-Alkylphenyl,
- R6, R⁷, R⁸, R⁹: Wasserstoff (H), Methyl oder Ethyl,
- X: CH₂, CHR⁵, Sauerstoff (O), Schwefel (S) oder NR⁵,
- Y: N(R¹⁰)₂, Hydroxy, C₂₋₂₀-Alkylaminoalkyl oder C₃₋₂₀-Dialkylaminoalkyl,
- n: eine ganze Zahl von 1 bis 30 und
- j, k, l, m, q: eine ganze Zahl von 1 bis 4,
bedeuten.

Das erfindungsgemäße Verfahren findet daher bevorzugt zur Herstellung eines Amins I Anwendung, indem man einen primären oder sekundären Alkohol der Formel II 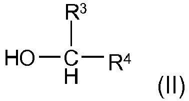 und/oder Aldehyd und/oder Keton der Formel VI bzw. VII 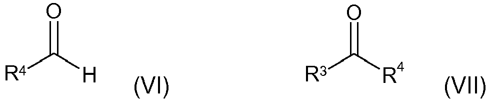 mit einer Stickstoffverbindung der Formel III 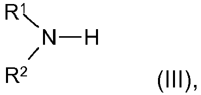 wobei R¹, R², R³ und R⁴ die oben genannten Bedeutungen haben, umsetzt.

Bei dem Eduktalkohol kann es sich auch um einen Aminoalkohol handeln, z. B. einem Aminoalkohol gemäß der Formel II.

Wie aus den Definitionen für die Reste R² und R⁴ hervorgeht, kann die Umsetzung auch intramolekular in einem entsprechenden Aminoalkohol, Aminoketon oder Aminoaldehyd erfolgen.
Zur Herstellung des Amins I wird demnach rein formal ein Wasserstoffatom der Stickstoffverbindung III durch den Rest R⁴(R³)CH- unter Freisetzung von einem Moläquivalent Wasser ersetzt.

Das erfindungsgemäße Verfahren findet auch bevorzugt Anwendung bei der Herstellung eines zyklischen Amins der Formel IV 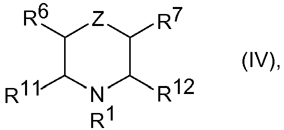 in der
- R¹¹ und R¹²: Wasserstoff (H), Alkyl, wie C₁- bis C₂₀-Alkyl, Cycloalkyl, wie C₃- bis C₁₂-Cycloalkyl, Aryl, Heteroaryl, Aralkyl, wie C₇- bis C₂₀-Aralkyl, und Alkylaryl, wie C₇- bis C₂₀-Alkylaryl,
- Z: CH₂, CHR⁵, Sauerstoff (O), NR⁵ oder NCH₂CH₂OH bedeuten und
- R¹, R⁶, R⁷: die oben genannten Bedeutungen haben,
durch Umsetzung eines Alkohols der Formel V 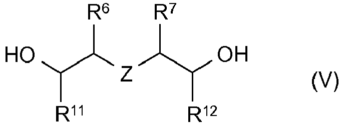 mit Ammoniak oder einem primären Amin der Formel VIII

R¹-NH₂ (VIII).

Die Substituenten R¹ bis R¹², die Variablen X, Y, Z und die Indizes j, k, l, m, n und q in den Verbindungen I, II, III, IV, V, VI und VII haben unabhängig voneinander folgende Bedeutungen:
R¹, R², R³, R⁴, R⁵, R⁶, R⁷, R⁸, R⁹, R¹⁰, R¹¹, R¹²:
   - Wasserstoff (H),
R³, R⁴:
   - Alkyl, wie C₁₋₂₀-Alkyl, bevorzugt C₁₋₁₄-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl,
   - Hydroxyalkyl, wie C₁₋₂₀-Hydroxyalkyl, bevorzugt C₁₋₈-Hydroxyalkyl, besonders bevorzugt C₁₋₄-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-(Hyd roxymethyl)ethyl,
   - Aminoalkyl, wie C₁₋₂₀-Aminoalkyl, bevorzugt C₁₋₈-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Amino-n-pentyl, N-(2-Aminoethyl)-2-aminoethyl und N-(2-Aminoethyl)aminomethyl,
   - Hydroxyalkylaminoalkyl, wie C₂₋₂₀-Hydroxyalkylaminoalkyl, bevorzugt C₃₋₈-Hydroxyalkylaminoalkyl, wie (2-Hydroxyethylamino)methyl, 2-(2-Hydroxyethylamino)ethyl und 3-(2-Hydroxyethylamino)propyl,
   - R⁵-(OCR⁶R⁷CR⁸R⁹)ₙ-(OCR⁶R⁷), bevorzugt R⁵-(OCHR⁷CHR⁹)ₙ-(OCR⁶R⁷), besonders bevorzugt R⁵-(OCH₂CHR⁹)ₙ-(OCR⁶R⁷),
   - Alkylaminoalkyl, wie C₂₋₃₀-Alkylaminoalkyl, bevorzugt C₂₋₂₀-Alkylaminoalkyl, besonders bevorzugt C₂₋₈-Alkylaminoalkyl, wie Methylaminomethyl, 2-Methylaminoethyl, Ethylaminomethyl, 2-Ethylaminoethyl und 2-(iso-Propylamino)ethyl, (R⁵)HN-(CH₂)_{q},
   - Y-(CH₂)ₘ-NR⁵-(CH₂)_{q},
   - Heteroarylalkyl, wie C₄₋₂₀-Heteroarylalkyl, wie Pyrid-2-yl-methyl, Furan-2-yl-methyl, Pyrrol-3-yl-methyl und Imidazol-2-yl-methyl,
   - Alkylheteroaryl, wie C₄₋₂₀-Alkylheteroaryl, wie 2-Methyl-3-pyridinyl, 4,5-Dimethyl-imidazol-2-yl, 3-Methyl-2-furanyl und 5-Methyl-2-pyrazinyl,
   - Heteroaryl, wie C₃₋₅-Heteroaryl mit mindestens einem Heteroatom aus N, O, wie 2-Pyridinyl, 3-Pyridinyl, 4-Pyridinyl, Pyrazinyl, Pyrrol-3-yl, Imidazol-2-yl, 2-Furanyl und 3-Furanyl,
R¹, R², R³, R⁴:
   - Cycloalkyl, wie C₃₋₁₂-Cycloalkyl, bevorzugt C₃₋₈-Cycloalkyl, wie Cyclopropyl, Cyclobutyl, Cyclopentyl, Cyclohexyl, Cycloheptyl und Cyclooctyl, besonders bevorzugt Cyclopentyl und Cyclohexyl,
   - Alkoxyalkyl, wie C₂₋₃₀-Alkoxyalkyl, bevorzugt C₂₋₂₀-Alkoxyalkyl, besonders bevorzugt C₂₋₈-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxyethyl, besonders bevorzugt C₂₋₄-Alkoxyalkyl,
   - Dialkylaminoalkyl, wie C₃₋₃₀-Dialkylaminoalkyl, bevorzugt C₃₋₂₀-Dialkylaminoalkyl, besonders bevorzugt C₃₋₁₀-Dialkylaminoalkyl, wie N,N-Dimethylaminomethyl, (N,N-Dibutylamino)methyl, 2-(N,N-Dimethylamino)ethyl, 2-(N,N-Diethylamino)ethyl, 2-(N,N-Dibutylamino)ethyl, 2-(N,N-Di-n-propylamino)ethyl und 2-(N,N-Di-iso-propylamino)ethyl, 3-(N,N-Dimethylamino)propyl, (R⁵)₂N-(CH₂)q,
   - Aryl, wie C₆₋₁₄-Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
   - Alkylaryl, wie C₇₋₂₀-Alkylaryl, bevorzugt C₇₋₁₂-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
   - Aralkyl, wie C₇₋₂₀-Aralkyl, bevorzugt C₇₋₁₂-Phenylalkyl, wie Benzyl, p-Methoxybenzyl, 3,4-Dimethoxybenzyl, 1-Phenethyl, 2-Phenethyl, 1-Phenyl-propyl, 2-Phenylpropyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
   - R³ und R⁴ oder R² und R⁴ gemeinsam eine -(CH₂)ₗ-X-(CH₂)ₘ- Gruppe, wie - (CH₂)₃-, -(CH₂)₄-, -(CH₂)₅-, -(CH₂)₆-, -(CH₂)₇-, -(CH₂)-O-(CH₂)₂-, -(CH₂)-NR⁵-(CH₂)₂-, -(CH₂)-CHR⁵-(CH₂)₂-, -(CH₂)₂-O-(CH₂)₂-, -(CH₂)₂-NR⁵-(CH₂)₂-, -(CH₂)₂-CHR⁵-(CH₂)₂-, -CH₂-O-(CH₂)₃-, -CH₂-NR⁵-(CH₂)₃-, -CH₂-CHR⁵-(CH₂)₃-,
R¹, R²:
   - Alkyl, wie C₁₋₂₀-Alkyl, bevorzugt C₁₋₈-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, 2-Ethylhexyl, besonders bevorzugt C₁₋₄-Alkyl, oder
   - R¹ und R² gemeinsam eine -(CH₂)ⱼ-X-(CH₂)ₖ- Gruppe, wie -(CH₂)₃-, -(CH₂)₄-, - (CH₂)₅-, -(CH₂)₆-, -(CH₂)₇-, -(CH₂)-O-(CH₂)₂-, -(CH₂)-NR⁵-(CH₂)₂-, -(CH₂)-CHR⁵-(CH₂)₂-, -(CH₂)₂-O-(CH₂)₂-, -(CH₂)₂-NR⁵-(CH₂)₂-, -(CH₂)₂-CHR⁵-(CH₂)₂-, -CH₂-O-(CH₂)₃-, -CH₂-NR⁵-(CH₂)₃-, -CH₂-CHR⁵-(CH₂)₃-,
R⁵, R¹⁰:
   - Alkyl, bevorzugt C₁₋₄-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
   - Alkylphenyl, bevorzugt C₇₋₄₀-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl, insbesondere C₇₋₂₀-Alkylphenyl,
R⁶, R⁷, R⁸, R⁹:
   - Methyl oder Ethyl, bevorzugt Methyl,
R¹¹, R¹²:
   - Alkyl, wie C₁- bis C₂₀-Alkyl, Cycloalkyl, wie C₃- bis C₁₂-Cycloalkyl, Aryl, Heteroaryl, Aralkyl, wie C₇- bis C₂₀-Aralkyl, und Alkylaryl, wie C₇- bis C₂₀-Alkylaryl, jeweils wie oben definiert,
X:
   - CH₂, CHR⁵, Sauerstoff (O), Schwefel (S) oder NR⁵, bevorzugt CH₂ und O,
Y:
   - N(R¹⁰)₂, bevorzugt NH₂ und N(CH₃)₂,
   - Hydroxy (OH),
   - C₂₋₂₀-Alkylaminoalkyl, bevorzugt C₂₋₁₆-Alkylaminoalkyl, wie Methylaminomethyl, 2-Methylaminoethyl, Ethylaminomethyl, 2-Ethylaminoethyl und 2-(iso-Propylamino)ethyl,
   - C₃₋₂₀-Dialkylaminoalkyl, bevorzugt C₃₋₁₆-Dialkylaminoalkyl, wie Dimethylaminomethyl, 2-Dimethylaminoethyl, 2-Diethylaminoethyl, 2-(Di-n-propylamino)ethyl und 2-(Di-iso-propylamino)ethyl,
Z:
   - CH₂, CHR⁵, O, NR⁵ oder NCH₂CH₂OH,
j, l:
   - eine ganze Zahl von 1 bis 4 (1, 2, 3 oder 4), bevorzugt 2 und 3, besonders bevorzugt 2,
k, m, q:
   - eine ganze Zahl von 1 bis 4 (1, 2, 3 oder 4), bevorzugt 2, 3 und 4, besonders bevorzugt 2 und 3,
n:
   - eine ganze Zahl von 1 bis 30, bevorzugt eine ganze Zahl von 1 bis 8 (1, 2, 3, 4, 5, 6, 7 oder 8), besonders bevorzugt eine ganze Zahl von 1 bis 6.

Als Alkohole eignen sich unter den o. g. Voraussetzungen praktisch alle primären und sekundären Alkohole mit aliphatischer OH-Funktion. Die Alkohole können geradkettig, verzweigt oder zyklisch sein. Sekundäre Alkohole werden ebenso aminiert wie primäre Alkohole. Die Alkohole können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Alkohole, wie z. B. Diole oder Triole, besonders Glykole, aminiert werden, so hat man es über die Steuerung der Reaktionsbedingungen in der Hand, bevorzugt Aminoalkohole, zyklische Amine oder mehrfach aminierte Produkte zu erhalten.

Die Aminierung von 1,2-Diolen führt je nach Wahl der Reaktionsbedingungen besonders zu 1-Amino-2-hydroxy- oder 1,2-Diamino-Verbindungen.

Die Aminierung von 1,4-Diolen führt je nach Wahl der Reaktionsbedingungen zu 1-Amino-4-hydroxy-, 1,4-Diamino-Verbindungen oder zu fünfgliedrigen Ringen mit einem Stickstoffatom (Pyrrolidine).

Die Aminierung von 1,6-Diolen führt je nach Wahl der Reaktionsbedingungen zu 1-Amino-6-hydroxy-, 1,6-Diamino-Verbindungen oder zu siebengliedrigen Ringen mit einem Stickstoffatom (Hexamethylenimine).

Die Aminierung von 1,5-Diolen führt je nach Wahl der Reaktionsbedingungen zu 1-Amino-5-hydroxy-, 1,5-Diamino-Verbindungen oder zu sechsgliedrigen Ringen mit einem Stickstoffatom (Piperidine, 1,5-Di-piperidinyl-pentane).
Aus Diglykol (DEG) kann demnach durch Aminierung mit NH₃ Monoaminodiglykol (= ADG = H₂N-CH₂CH₂-O-CH₂CH₂-OH), Diaminodiglykol (H₂N-CH₂CH₂-O-CH₂CH₂-NH₂) oder Morpholin erhalten werden. Besonders bevorzugt ist hier das ADG als Verfahrensprodukt.
Aus Diethanolamin wird entsprechend besonders bevorzugt Piperazin erhalten. Aus Triethanolamin kann N-(2-Hydroxyethyl)-piperazin erhalten werden.

Bevorzugt werden beispielsweise die folgenden Alkohole aminiert:
Methanol, Ethanol, n-Propanol, Isopropanol, n-Butanol, iso-Butanol, n-Pentanol, n-Hexanol, 2-Ethylhexanol, Tridecanol, Stearylalkohol, Palmitylalkohol, Cyclobutanol, Cyclopentanol, Cyclohexanol, Benzylalkohol, 2-Phenyl-ethanol, 2-(p-Methoxyphenyl)-ethanol, 2-(3,4-Dimethoxyphenyl)ethanol, 1-Phenyl-3-butanol, Ethanolamin, n-Propanolamin, Isopropanolamin, 2-Amino-1-propanol, 1-Methoxy-2-propanol, 3-Amino-2,2-dimethyl-1-propanol, n-Pentanolamin (1-Amino-5-pentanol), n-Hexanolamin (1-Amino-6-hexanol), Ethanolamin, Diethanolamin, Triethanolamin, N-Alkyldiethanolamine, Diisopropanolamin, 3-(2-Hydroxyethylamino)propan-1-ol, 2-(N,N-Dimethylamino)ethanol, 2-(N,N-Diethylamino)ethanol, 2-(N,N-Di-n-propylamino)ethanol, 2-(N,N-Di-iso-propylamino)ethanol, 2-(N,N-Di-n-butylamino)ethanol, 2-(N,N-Di-iso-butylamino)ethanol, 2-(N,N-Di-sec.-butylamino)ethanol, 2-(N,N-Di-tert.-butyl-amino)ethanol, 3-(N,N-Dimethylamino)propanol, 3-(N,N-Diethylamino)propanol, 3-(N,N-Di-n-propylamino)propanol, 3-(N,N-Di-iso-propylamino)propanol, 3-(N,N-Di-n-butylamino)propanol, 3-(N,N-Di-iso-butylamino)propanol, 3-(N,N-Di-sec.-butylamino)-propanol, 3-(N,N-Di-tert.-butylamino)propanol, 1-Dimethylamino-pentanol-4, 1-Diethylamino-pentanol-4, Ethylenglykol, 1,2-Propylenglykol, 1,3-Propylenglykol, Diglykol, 1,4-Butandiol, 1,5-Pentandiol, 1,6-Hexandiol, 2,2-Bis[4-hydroxycyclohexyl]propan, Methoxyethanol, Propoxyethanol, Butoxyethanol, Polypropylalkohole, Polyethylenglykolether, Polypropylenglykolether und Polybutylenglykolether. Die letztgenannten Polyalkylenglykolether werden bei der erfindungsgemäßen Umsetzung durch Umwandlung ihrer freien Hydroxylgruppen zu den entsprechenden Aminen umgewandelt.

Besonders bevorzugte Alkohole sind Methanol, Ethanol, n-Propanol, i-Propanol, n-Butanol, sek.-Butanol, 1,4-Butandiol, 1,5-Pentandiol, 1,6-Hexandiol, 2-Ethylhexanol, Cyclohexanol, Fettalkohole, Ethylenglykol, Diethylenglykol (DEG), Triethylenglykol (TEG), 2-(2-Dimethylamino-ethoxy)ethanol, N-Methyldiethanolamin und 2-(2-Dimethylaminoethoxy)ethanol.

Als im erfindungsgemäßen Verfahren einsetzbare Ketone eignen sich unter den o. g. Voraussetzungen praktisch alle aliphatischen und aromatischen Ketone. Die aliphatischen Ketone können geradkettig, verzweigt oder zyklisch sein, die Ketone können Heteroatome enthalten. Die Ketone können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Ketone aminiert werden, so hat man es über die Steuerung der Reaktionsbedingungen in der Hand, Aminoketone, Aminoalkohole, cyclische Amine oder mehrfach aminierte Produkte zu erhalten.

Bevorzugt werden beispielsweise die folgenden Ketone aminierend hydriert:
Aceton, Ethylmethylketon, Methylvinylketon, Isobutylmethylketon, Butanon, 3-Methylbutan-2-on, Diethylketon, Tetraion, Acetophenon, p-Methyl-acetophenon, p-Methoxy-acetophenon, m-Methoxy-acetophenon, 1-Acetyl-naphthalin, 2-Acetyl-naphthalin, 1-Phenyl-3-butanon, Cyclobutanon, Cyclopentanon, Cyclopentenon, Cyclohexanon, Cyclohexenon, 2,6-Dimethylcyclohexanon, Cycloheptanon, Cyclododecanon, Acetylaceton, Methylglyoxal und Benzophenon.

Als im erfindungsgemäßen Verfahren einsetzbare Aldehyde eignen sich unter den o. g. Voraussetzungen praktisch alle aliphatischen und aromatischen Aldehyde. Die aliphatischen Aldehyde können geradkettig, verzweigt oder zyklisch sein, die Aldehyde können Heteroatome enthalten. Die Aldehyde können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Aldehyde oder Ketoaldehyde aminiert werden, so hat man es über die Steuerung der Reaktionsbedingungen in der Hand, Aminoalkohole, cyclische Amine oder mehrfach aminierte Produkte zu erhalten.

Bevorzugt werden beispielsweise die folgenden Aldehyde aminierend hydriert:
Formaldehyd, Acetaldehyd, Propionaldehyd, n-Butyraldehyd, Isobutyraldehyd, Pivalinaldehyd, n-Pentanal, n-Hexanal, 2-Ethylhexanal, 2-Methylpentanal, 3-Methylpentanal, 4-Methylpentanal, Glyoxal, Benzaldehyd, p-Methoxybenzaldehyd, p-Methylbenzaldehyd, Phenylacetaldehyd, (p-Methoxy-phenyl)acetaldehyd, (3,4-Dimethoxyphenyl)-acetaldehyd, 4-Formyltetrahydropyran, 3- Formyltetrahydrofuran, 5-Formylvaleronitril, Citronellal, Lysmeral, Acrolein, Methacrolein, Ethylacrolein, Citral, Crotonaldehyd, 3-Methoxypropionaldehyd, 3-Aminopropionaldehyd, Hydroxypivalinaldehyd, Dimethylolpropionaldehyd, Dimethylolbutyraldehyd, Furfural, Glyoxal, Glutaraldehyd sowie hydroformylierte Oligomere und Polymere, wie z. B. hydroformyliertes Polyisobuten (Polyisobutenaldehyd) oder durch Metathese von 1-Penten und Cyclopenten erhaltenes und hydroformyliertes Oligomer.

Als Aminierungsmittel bei der hydrierenden Aminierung von Alkoholen, Aldehyden oder Ketonen in Gegenwart von Wasserstoff können sowohl Ammoniak als auch primäre oder sekundäre, aliphatische oder cycloaliphatische oder aromatische Amine eingesetzt werden.
Bei Verwendung von Ammoniak als Aminierungsmittel wird die alkoholische Hydroxylgruppe bzw. die Aldehydgruppe bzw. die Ketogruppe zunächst in die primäre Aminogruppen (-NH₂) umgewandelt. Das so gebildete primäre Amin können mit weiterem Alkohol bzw. Aldehyd bzw. Keton zu dem entsprechenden sekundären Amin und diese wiederum mit weiterem Alkohol bzw. Aldehyd bzw. Keton zu dem entsprechenden, vorzugsweise symmetrischen, tertiären Amin reagieren. Je nach Zusammensetzung des Reaktionsansatzes oder des Eduktstroms (bei kontinuierlicher Fahrweise) und je nach den angewandten Reaktionsbedingungen - Druck, Temperatur, Reaktionszeit (Katalysatorbelastung) - lassen sich auf diese Weise je nach Wunsch bevorzugt primäre, sekundäre oder tertiäre Amine darstellen.

Aus mehrwertigen Alkoholen bzw. Di- oder Oligoaldehyden bzw. Di- oder Oligoketonen bzw. Ketoaldehyden lassen sich auf diese Weise durch intramolekulare hydrierende Aminierung zyklische Amine wie z. B. Pyrrolidine, Piperidine, Hexamethylenimine, Piperazine und Morpholine herstellen.

Ebenso wie Ammoniak lassen sich primäre oder sekundäre Amine als Aminierungsmittel verwenden.

Bevorzugt werden diese Aminierungsmittel zur Herstellung unsymmetrisch substituierter Di- oder Trialkylamine, wie Ethyldiisopropylamin und Ethyldicyclohexylamin verwendet. Beispielsweise werden die folgenden Mono- und Dialkylamine als Aminierungsmittel verwendet: Monomethylamin, Dimethylamin, Monoethylamin, Diethylamin, n-Propylamin, Di-n-propyl-amin, iso-Propylamin, Di-isopropyl-amin, Isopropylethylamin, n-Butylamin, Di-n-Butylamin, s-Butylamin, Di-s-Butylamin, iso-Butylamin, n-Pentylamin, s-Pentylamin, iso-Pentylamin, n-Hexylamin, s-Hexylamin, iso-Hexylamin, Cyclohexylamin, Anilin, Toluidin, Piperidin, Morpholin und Pyrrolidin.

Mit dem erfindungsgemäßen Verfahren besonders bevorzugt hergestellte Amine sind zum Beispiel Morpholin (aus Monoaminodiglykol), Monoaminodiglykol, Morpholin und/oder 2,2'-Dimorpholinodiethylether (DMDEE) (aus DEG und Ammoniak), 6-Dimethylaminohexanol-1 (aus Hexandiol und Dimethylamin (DMA)), Triethylamin (aus Ethanol und Diethylamin (DEA)), Dimethylethylamin (aus Ethanol und DMA), N-(C₁₋₄-alkyl)morpholin (aus DEG und Mono(C₁₋₄-alkyl)amin), N-(C₁₋₄-alkyl)piperidin (aus 1,5-Pentandiol und Mono(C₁-₄-alkyl)amin), Piperazin und/oder Diethylentriamin (DETA) (aus N-(2-Aminoethyl)-ethanolamin (AEEA) und Ammoniak), N-Methylpiperazin (aus Diethanolamin und MMA), N,N'-Dimethylpiperazin (aus N-Methyldiethanolamin und MMA), 1,2-Ethylendiamin (EDA) und/oder Diethylentriamin (DETA) und/oder PIP (aus Monoethanolamin (MEOA) und Ammoniak), 2-Ethylhexylamin und Bis(2-Ethylhexyl)amin (aus 2-Ethylhexanol und NH₃), Tridecylamin und Bis(Tridecyl)amin (aus Tridecanol und NH₃), n-Octylamin (aus n-Octanol und NH₃), 1,2-Propylendiamin (aus 2-Hydroxy-propylamin und NH₃), 1-Diethylamino-4-aminopentan (aus 1-Diethylamino-4-hydroxypentan und NH₃), N,N-Di(C₁₋₄-alkyl)cyclohexylamin (aus Cyclohexanon und/oder Cyclohexanol und Di(C₁₋₄-alkyl)amin), z. B. N,N-Dimethyl-N-cyclohexylamin (DMCHA), Polyisobutenamin (PIBA; mit z. B. n~1000) (aus Polyisobutenaldehyd und NH₃), N,N-Diisopropyl-N-ethylamin (Hünigbase) (aus N,N-Diisopropylamin und Acetaldehyd), N-Methyl-N-isopropylamin (MMIPA) (aus Monomethylamin und Aceton), n-Propylamine (wie Mono-/Di-n-propylamin, N,N-Dimethyl-N-n-propylamin (DMPA)) (aus Propionaldehyd und/oder n-Propanol und NH₃ bzw. DMA), N,N-Dimethyl-N-isopropylamin (DMIPA) (aus i-Propanol und/oder Aceton und DMA), N,N-Dimethyl-N-butylamine (1-, 2- oder iso-Butanol und/oder Butanal, i-Butanal oder Butanon und DMA), 2-(2-Di(C₁₋₄-alkyl)aminoethoxy)ethanol und/oder Bis(2-di(C₁₋₄-alkyl)aminoethyl)ether (aus DEG und Di(C₁₋₄-alkyl)amin), 1,2-Ethylendiamin (EDA), Monoethanolamin (MEOA), Diethylentriamin (DETA) und/oder Piperazin (PIP) (aus Monoethylenglykol (MEG) und Ammoniak), 1,8-Diamino-3,6-dioxa-octan und/oder 1-Amino-8-hydroxy-3,6-dioxa-octan (aus Triethylenglykol (TEG) und Ammoniak), 1-Methoxy-2-propylamin (1-Methoxy-isopropylamin, MOIPA) (aus 1-Methoxy-2-propanol und Ammoniak), N-Cyclododecyl-2,6-dimethylmorpholin (Dodemorph) (aus Cyclododecanon und/oder Cyclododecanol und 2,6-Dimethylmorpholin), Polyetheramin (aus entsprechendem Polyetheralkohol und Ammoniak). Die Polyetheralkohole sind z. B. Polyethylenglykole oder Polypropylenglykole mit einem Molekulargewicht im Bereich von 200 bis 5000 g/mol, die entsprechende Polyetheramine sind z. B. unter dem Handelsname PEA D230, D400, D2000, T403 oder T5000 von BASF erhältlich.

Alle Druckangaben beziehen sich auf den Absolutdruck.

### Beispiele

### Vergleichsbeispiel 1

### Herstellung eines Aminierungskatalysators auf Basis von Ni-Co-Cu/ZrO₂ (Vergleichsversuch nach EP 963 975 A)

Eine wässrige Lösung aus Nickelnitrat, Kobaltnitrat, Kupfernitrat und Zirkoniumacetat, die 2,39 Gew.-% NiO, 2,39 Gew.-% CoO, 0,94 Gew.-% CuO und 2,82 Gew.-% ZrO₂ enthielt, wurde gleichzeitig in einem Rührgefäß in einem konstanten Strom mit einer 20 Gew.-%igen wässrigen Natriumcarbonatlösung bei einer Temperatur von 70 °C so gefällt, dass der mit einer Glaselektrode gemessene pH-Wert von 7,0 aufrechterhalten wurde. Die erhaltene Suspension wurde filtriert und der Filterkuchen mit voll entsalztem Wasser gewaschen bis die elektrische Leitfähigkeit des Filtrats ca. 20 µS betrug. Danach wurde der Filterkuchen bei einer Temperatur von 150 °C in einem Trockenschrank oder einem Sprühtrockner getrocknet. Das auf diese Weise erhaltene Hydroxid-Carbonat-Gemisch wurde nun bei einer Temperatur von 450 bis 500 °C über einen Zeitraum von 4 Stunden calciniert. Der so hergestellte Katalysator hatte die Zusammensetzung: 28 Gew.-% NiO, 28 Gew.-% CoO, 11 Gew.-% CuO und 33 Gew.-% ZrO₂. Der Katalysator wurde mit 3 Gew.-% Graphit vermischt und zu Tabletten verformt. Die oxidischen Tabletten wurden reduziert. Die Reduktion wurde bei 280 °C durchgeführt, wobei die Aufheizungsrate 3 °C/Minute betrug. Zuerst wurde 50 Minuten mit 10 % H₂ in N₂ reduziert, anschließend 20 Minuten mit 25 % H₂ in N₂, dann 10 Minuten mit 50 % H₂ in N₂, dann 10 Minuten mit 75 % H₂ in N₂ und schließlich 3 Stunden mit 100 % H₂. Bei den %-Angaben handelt es sich jeweils um Volumen-%. Die Passivierung des reduzierten Katalysator wurde bei Raumtemperatur in verdünnter Luft (Luft in N₂ mit einem O₂-Gehalt von maximal 5 Vol.-%) durchgeführt.

### Vergleichsbeispiel 2

### Herstellung eines Aminierungskatalysators auf Basis von Ni-Cu-Mo/ZrO₂ (Vergleichsversuch nach EP 696 572 A)

Eine wässrige Lösung aus Nickelnitrat, Kupfernitrat und Zirkoniumacetat, die 4,48 Gew.-% Ni (berechnet als NiO), 1,52 Gew.-% Cu (berechnet als CuO und 2,28 Gew.-% Zr (berechnet als ZrO₂) enthielt, wurde gleichzeitig in einem Rührgefäß in einem konstanten Strom mit einer 20 Gew.-%igen wässrigen Natriumcarbonatlösung bei einer Temperatur von 70°C so gefällt, dass der mit einer Glaselektrode gemessene pH-Wert von 7,0 aufrechterhalten wurde. Die erhaltene Suspension wurde filtriert und der Filterkuchen mit voll entsalztem Wasser gewaschen bis die elektrische Leitfähigkeit des Filtrats ca. 20 µS betrug. Danach wurde in den noch feuchten Filterkuchen 12,9 g Ammoniumheptamolybdat pro 50 g Nickelsalz, berechnet als NiO, eingearbeitet, so dass das nachfolgend angegebene Oxidgemisch erhalten wurde. Anschließend wurde der Filterkuchen bei einer Temperatur von 150°C in einem Trockenschrank oder einem Sprühtrockner getrocknet. Das getrocknete Hydroxid-Carbonat-Gemisch wurde nachfolgend bei einer Temperatur von 430 bis 460°C über einen Zeitraum von 4 Stunden calciniert. Der so hergestellte Katalysator wies folgende Zusammensetzung auf: 50 Gew.-% NiO, 17 Gew.-% CuO, 1,5 Gew.-% MoO₃ und 31,5 Gew.-% ZrO₂.

Der so erhaltene Katalysator wurde mit 3 Gew.-% Graphit versetzt, kompaktiert, und schließlich zu Tabletten verformt. Die Tabletten wurden anschließend reduziert. Die Reduktion wurde bei 290°C mit einem Gemisch bestehend aus 20 Vol.-% Wasserstoff und 80 Vol.-% Stickstoff durchgeführt, wobei die Aufheizungsrate 3°C/Minute betrug. Die Passivierung der reduzierten Katalysators wurde bei Raumtemperatur in verdünnter Luft (Luft in N₂ mit einem O₂-Gehalt von maximal 5 Vol.-%) durchgeführt.

### Vergleichsbeispiel 3

### Herstellung eines Aminierungskatalysators auf Basis von Ni-Co-Cu-Sn/ZrO₂ (Vergleichsversuch nach WO 2008/006750 A1)

Der Katalysator wurde analog Vergleichsbeispiel 1 hergestellt. Allerdings wurde die Menge des Nickelnitrats, Kupfernitrats und Kobaltnitrats entsprechend verändert und der Nitratlösung noch zusätzlich Zinndichlorid zugefügt.

Das auf der oben genannten Weise erhaltene Hydroxid-Carbonat-Gemisch wurde bei einer Temperatur 450°C über einen Zeitraum von 4 Stunden calciniert. Die so erhaltene Masse wurde mit 3 Gew.-% Graphit versetzt, kompaktiert, und schließlich zu Tabletten verformt. Die Tabletten wurden anschließend reduziert. Die Reduktion wurde bei 290°C mit einem Gemisch bestehend aus 20 Vol.-% Wasserstoff und 80 Vol.-% Stickstoff durchgeführt, wobei die Aufheizungsrate 3°C/Minute betrug. Die Passivierung der reduzierten Katalysators wurde bei Raumtemperatur in verdünnter Luft (Luft in N₂ mit einem O₂-Gehalt von maximal 5 Vol.-%) durchgeführt. Der so erhaltene Katalysator hatte die Zusammensetzung wie in der folgenden Tabelle I dargestellt.

### Beispiel 4 (erfindungsgemäß)

Eine wässrige Lösung aus Nickelnitrat, Kobaltnitrat, Kupfernitrat, Aluminiumnitrat, und Zinn(II)chlorid, die 3,9 % Ni, 3,9 % Co, 1,9 % Cu, 5,5 % Al₂O₃ und 0,5 % Sn enthielt, wurde gleichzeitig in einem Rührgefäß in einem konstanten Strom mit einer 20 Gew.-%igen wässrigen Natriumcarbonatlösung bei einer Temperatur von 65-70 °C so gefällt, dass der mit einer Glaselektrode gemessene pH-Wert von 5,7 aufrechterhalten wurde. Nach der Fällung wurde für 1 Stunde Luft eingeblasen, danach wurde das pH des Lösungs mit Natriumcarbonatlösung auf den Wert 7,4 eingestellt. Die erhaltene Suspension wurde filtriert und der Filterkuchen mit vollentsalztem Wasser gewaschen bis die elektrische Leitfähigkeit des Filtrats ca. 20 mS betrug. Danach wurde der Filterkuchen bei einer Temperatur von 150 °C in einem Trockenschrank getrocknet. Das auf diese Weise erhaltene Hydroxidcarbonatgemisch wurde nun bei einer Temperatur von 500 °C über 4 Stunden calziniert. Die Katalysatormasse wurde anschließend mit 3 Gew.-% Graphit vermischt und zu Tabletten 3x3 mm verformt. Die auf dieser Weise erhaltene Tabletten werden bei einer Temperatur von 280-300 °C über mindestens 12 Stunden in Wasserstoff reduziert. Die Passivierung des reduzierten Katalysators wurde bei Raumtemperatur in verdünnter Luft (Luft in N₂ mit einem O₂-Gehalt von maximal 5 Vol.-%) durchgeführt. Der so erhaltene Katalysator hatte die Zusammensetzung wie in der folgenden Tabelle I dargestellt.

### Beispiel 5 (erfindungsgemäß)

Der Katalysator wurde analog Vergleichsbeispiel 3 hergestellt. Allerdings wurde die Menge des Nickelnitrats, Kupfernitrats, Kobaltnitrats und Zinnchlorids entsprechend verändert. Anstelle von Zirkoniumacetat-Lösung wurde fein dispergiertes Aluminiumoxid-Pulver (D10-10 von BASF SE) eingerührt.

Das auf der oben genannten Weise erhaltene Hydroxid-Carbonat-Gemisch wurde bei einer Temperatur 450 °C über einen Zeitraum von 4 Stunden calciniert. Die so erhaltene Masse wurde mit 3 Gew.-% Graphit versetzt, kompaktiert, und schließlich zu Tabletten verformt. Die Tabletten wurden anschließend reduziert. Die Reduktion wurde bei 290 °C mit einem Gemisch bestehend aus 20 Vol.-% Wasserstoff und 80 Vol.-% Stickstoff durchgeführt, wobei die Aufheizungsrate 3 °C/Minute betrug. Die Passivierung der reduzierten Katalysators wurde bei Raumtemperatur in verdünnter Luft (Luft in N₂ mit einem O₂-Gehalt von maximal 5 Vol.-%) durchgeführt. Der so erhaltene Katalysator hatte die Zusammensetzung wie in der folgenden Tabelle I dargestellt.

### Durchführung der Katalysetests im kontinuierlich betriebenen Rohrreaktor Aminierung von Diethylenglykol (DEG)

Ein beheizter Rohrreaktor mit 14 mm Innendurchmesser, einem zentral angebrachten Thermoelement und einem Gesamtvolumen von 89 ml wurde im unteren Teil mit einer Schicht Glaskugeln (15 ml) befüllt, darüber mit 30 ml des reduzierten Aminierungskatalysators (in Form vom ca. 1,0 bis 1,6 mm Splitt, der aus den reduzierten und passivierten Tabletten hergestellt wurde) und schließlich der restliche Teil wiederum mit Glaskugeln befüllt. Vor der Reaktion wurde der Katalysator bei max. 280 °C unter Wasserstoff (25 Nl/h) (Nl = Normliter = auf Normalbedingungen (20 °C, 1 bar abs.) umgerechnetes Volumen) bei Normaldruck 24 Stunden aktiviert. Durch den Reaktor wurden von unten nach oben 35 g/h DEG, 35 g/h flüssiger NH₃ und 7 Nl/h Wasserstoff dosiert. Der Reaktor wurde bei einer Temperatur von ca. 190 bis 210 °C und einem Gesamtdruck von 200 bar gehalten. Die Reaktionstemperatur wurde so gewählt, dass ein DEG Umsatz von ca. 65-70 % erreicht wurde. Das aus dem Reaktor austretende Gemisch wurde abgekühlt und auf Normaldruck entspannt. Zu verschiedenen Zeitpunkten wurden Proben vom Reaktionsgemisch genommen und mittels Gaschromatographie analysiert. Hierfür wurde eine 30 m lange GC Säule "RTX-5 Amine" verwendet, mit einem Temperaturprogramm: 80 °C/15 Minuten, aufheizen auf 290 °C in 30 Minuten, bei 290 °C/15 Minuten.

Die Zusammensetzung der resultierenden Reaktionsgemische für die Katalysatoren der Beispiele 1 bis 5 ist der folgenden Tabelle II zu entnehmen.

**Tabelle I**

| Katalysator *) | Ni | Co | Cu | Sn | Mo | BET **) | Träger |
|---|---|---|---|---|---|---|---|
| | % | % | % | % | % | m²/g | |
| Vergleichsbsp. 1 | 21,9 | 21,9 | 10,5 | - | - | 90 | ZrO2 |
| Vergleichsbsp. 2 | 45,0 | - | 12,0 | - | 1,5 | 85 | ZrO2 |
| Vergleichsbsp. 3 | 18,7 | 18,0 | 10,2 | 1,0 | - | 62 | ZrO2 |
| Beispiel 4 | 18,6 | 17,3 | 10,6 | 1,1 | - | 187 | Al2O3 |
| Beispiel 5 | 17,6 | 17,3 | 9,7 | 0,9 | - | 154 | Al2O3 |

| | | | | | | | |
|---|---|---|---|---|---|---|---|
| *) Katalysatorzusammensetzung in Gew.-%; Rest bis zu 100 Gew.-% ist der Träger **) ISO 9277:1995 | | | | | | | |

**Tabelle II:**

| Katalysator | Kat. Menge | | TOS | Temp. | Bel. | MV | DEG Umsatz | ADG | MOR | Verhältnis | Gessel. | Methoxy | ES |
|---|---|---|---|---|---|---|---|---|---|---|---|---|---|
| | ml | g | h | °C | kg/lKat.•h | | mol% | mol% | mol% | ADG/MOR | mol% | GC% | GC % |
| Vergleichsbsp. 1 | 30 | 37,7 | 142 | 190 | 1,2 | 6,2 | 72,5 | 40,2 | 47,3 | 0,85 | 89,2 | 0,230 | 0,62 |
| | | | 246 | 190 | 1,2 | 6,2 | 70,7 | 39,9 | 48,2 | 0,83 | 89,8 | 0,220 | 0,57 |
| Vergleichsbsp. 2 | 30 | 36,8 | 136 | 193 | 1,2 | 6,2 | 69,4 | 55,6 | 32,8 | 1,69 | 90,2 | 0,170 | 1,17 |
| | | | 236 | 193 | 1,2 | 6,2 | 69,2 | 55,3 | 33,5 | 1,65 | 90,7 | 0,151 | 1,00 |
| Vergleichsbsp. 3 | 27 | 27,5 | 186 | 193 | 1,0 | 6,2 | 71,8 | 46,1 | 42,5 | 1,08 | 91,4 | 0,061 | 0,31 |
| | | | 282 | 193 | 1,0 | 6,2 | 70,4 | 47,2 | 41,6 | 1,13 | 91,6 | 0,059 | 0,27 |
| Beispiel 4 | 30 | 20,7 | 75 | 198 | 1,2 | 6,2 | 75,1 | 52,4 | 37,5 | 1,40 | 93,0 | 0,050 | 0,14 |
| | | | 236 | 198 | 1,2 | 6,2 | 66,7 | 60,4 | 29,7 | 2,03 | 93,1 | 0,030 | 0,15 |
| | | | 356 | 198 | 1,2 | 6,2 | 58,1 | 65,6 | 25,2 | 2,60 | 93,4 | 0,030 | 0,13 |
| Beispiel 5 | 30 | 24,8 | 141 | 208 | 1,2 | 6,2 | 70,9 | 61,0 | 31,1 | 1,96 | 96,6 | 0,079 | 0,32 |
| | | | 196 | 208 | 1,2 | 6,2 | 67,6 | 65,1 | 26,9 | 2,42 | 96,5 | 0,065 | 0,31 |

| | | | | | | | | | | | | | |
|---|---|---|---|---|---|---|---|---|---|---|---|---|---|
| TOS: time on stream (Stunden) Temp.: Temperatur im Reaktor Bel.: Katalysatorbelastung (kg DEG/LiterKat•h) MV: Molverhältnis Ammoniak / DEG im Feed Gessel.: Gesamtselektivität; Summe ADG+MOR+Di-ADG (mol% Methoxy: Methoxyethanol im Rohaustrag (GC%) ES: Etherspaltung Komponente; Summe Ethanol, Ethylamin, Ethylmorpholin im Rohaustrag (GC%) | | | | | | | | | | | | | |

### Aufarbeitung:

Die jeweiligen Reinprodukte können aus den wasserhaltigen Rohwaren durch Rektifikation bei Vakuum, Normaldruck oder erhöhtem Druck nach den bekannten Methoden erhalten werden. Die Reinprodukte fallen dabei entweder direkt in reiner Form an oder als Azeotrope mit Wasser. Wasserhaltige Azeotrope können durch eine Flüssig-flüssig-Extraktion mit konzentrierter Natronlauge vor oder nach der Reindestillation entwässert werden. Eine destillative Entwässerung in Gegenwart eines Schleppmittels nach bekannten Methoden ist auch möglich.

Für den Fall, dass die Rohware oder das aliphatische Amin in der Rohware kaum oder nicht mit Wasser mischbar sind, ist eine Entwässerung durch eine Trennung der organischen und der wässrigen Phase mit bekannten Methoden auch möglich.

### Fazit:

Die Performance von Aminierungskatalysatoren konnte gegenüber dem Stand der Technik, unter Erhalt der guten Katalysatoraktivität, deutlich verbessert werden, indem man die chemische Zusammensetzung der Aktivmasse erfindungsgemäß veränderte.

Die Ausbeute an wirtschaftlich interessanten Aminierungsprodukten, wie Aminodiglykol und Morpholin bei der DEG-Aminierung, kann dadurch erhöht werden, dass ein entsprechender Katalysator mit Al₂O₃ als Träger in Kombination mit Ni, Co, Cu und Sn eingesetzt wird. Insbesondere kann dabei die Ausbeute an wertvollen linearen Aminierungsprodukten, wie Aminodiglykol bei der DEG-Aminierung, gesteigert werden. Weiterhin kann das Ausmaß der unerwünschten Decarbonylierung, das bei der DEG-Aminierung durch den Gehalt an Methoxyethanol bestimmt wird, und der Etherspaltung deutlich verringert werden.

## Patentansprüche

1. Verfahren zur Herstellung eines Amins durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, in Gegenwart eines geträgerten, kupfer-, nickel- und kobalthaltigen Katalysators, **dadurch gekennzeichnet, dass** die katalytisch aktive Masse des Katalysators vor dessen Reduktion mit Wasserstoff sauerstoffhaltige Verbindungen des Aluminiums, Kupfers, Nickels und Kobalts und im Bereich von 0,2 bis 5,0 Gew.-% sauerstoffhaltige Verbindungen des Zinns, berechnet als SnO, enthält.

2. Verfahren nach Anspruch 1, **dadurch gekennzeichnet, dass** die katalytisch aktive Masse des Katalysators vor dessen Reduktion mit Wasserstoff im Bereich von 0,4 bis 4,0 Gew.-% sauerstoffhaltige Verbindungen des Zinns, berechnet als SnO, enthält.

3. Verfahren nach Anspruch 1, **dadurch gekennzeichnet, dass** die katalytisch aktive Masse des Katalysators vor dessen Reduktion mit Wasserstoff im Bereich von 0,6 bis 3,0 Gew.-% sauerstoffhaltige Verbindungen des Zinns, berechnet als SnO, enthält.

4. Verfahren nach einem der vorhergehenden Ansprüche, **dadurch gekennzeichnet, dass** die katalytisch aktive Masse des Katalysators vor dessen Reduktion mit Wasserstoff im Bereich von 5,0 bis 35 Gew.-% sauerstoffhaltige Verbindungen des Kobalts, berechnet als CoO, enthält.

5. Verfahren nach einem der vorhergehenden Ansprüche, **dadurch gekennzeichnet, dass** die katalytisch aktive Masse des Katalysators vor dessen Reduktion mit Wasserstoff im Bereich von 10 bis 30 Gew.-% sauerstoffhaltige Verbindungen des Kobalts, berechnet als CoO, enthält.

6. Verfahren nach einem der vorhergehenden Ansprüche, **dadurch gekennzeichnet, dass** die katalytisch aktive Masse des Katalysators vor dessen Reduktion mit Wasserstoff im Bereich von
15 bis 80 Gew.-% sauerstoffhaltige Verbindungen des Aluminiums, berechnet als Al₂O_{3,}
1,0 bis 20 Gew.-% sauerstoffhaltige Verbindungen des Kupfers, berechnet als CuO, und
5,0 bis 35 Gew.-% sauerstoffhaltige Verbindungen des Nickels, berechnet als NiO, enthält.

7. Verfahren nach einem der Ansprüche 1 bis 5, **dadurch gekennzeichnet, dass** die katalytisch aktive Masse des Katalysators vor dessen Reduktion mit Wasserstoff im Bereich von
30 bis 70 Gew.-% sauerstoffhaltige Verbindungen des Aluminiums, berechnet als Al₂O₃,
2,0 bis 18 Gew.-% sauerstoffhaltige Verbindungen des Kupfers, berechnet als CuO, und
10 bis 30 Gew.-% sauerstoffhaltige Verbindungen des Nickels, berechnet als NiO, enthält.

8. Verfahren nach einem der vorhergehenden Ansprüche, **dadurch gekennzeichnet, dass** im Katalysator das Molverhältnis von Nickel zu Kupfer größer 1 ist.

9. Verfahren nach einem der vorhergehenden Ansprüche, **dadurch gekennzeichnet, dass** die katalytisch aktive Masse des Katalysators kein Rhenium und/oder Ruthenium enthält.

10. Verfahren nach einem der vorhergehenden Ansprüche, **dadurch gekennzeichnet, dass** die katalytisch aktive Masse des Katalysators kein Eisen und/oder Zink enthält.

11. Verfahren nach einem der vorhergehenden Ansprüche, **dadurch gekennzeichnet, dass** die katalytisch aktive Masse des Katalysators keine sauerstoffhaltigen Verbindungen des Siliziums und/oder des Zirkoniums enthält.

12. Verfahren nach einem der vorhergehenden Ansprüche, **dadurch gekennzeichnet, dass** die BET-Oberfläche des Katalysators (ISO 9277:1995) im Bereich von 30 bis 250 m²/g beträgt.

13. Verfahren nach einem der vorhergehenden Ansprüche, **dadurch gekennzeichnet, dass** man die Umsetzung bei einer Temperatur im Bereich von 80 bis 350 °C durchführt.

14. Verfahren nach einem der vorhergehenden Ansprüche, **dadurch gekennzeichnet, dass** man die Umsetzung in der Flüssigphase bei einem Absolutdruck im Bereich von 5 bis 30 MPa oder in der Gasphase bei einem Absolutdruck im Bereich von 0,1 bis 40 MPa durchführt.

15. Verfahren nach einem der vorhergehenden Ansprüche, **dadurch gekennzeichnet, dass** die Aminkomponente (Stickstoffverbindung) in der 0,90- bis 100-fachen molaren Menge bezogen auf den/das eingesetzte/n Alkohol, Aldehyd und/oder Keton eingesetzt wird.

16. Verfahren nach einem der vorhergehenden Ansprüche, **dadurch gekennzeichnet, dass** der Katalysator im Reaktor als Festbett angeordnet ist.

17. Verfahren nach einem der vorhergehenden Ansprüche, **dadurch gekennzeichnet, dass** es kontinuierlich durchgeführt wird.

18. Verfahren nach dem vorhergehenden Anspruch, **dadurch gekennzeichnet, dass** die Umsetzung in einem Rohrreaktor erfolgt.

19. Verfahren nach einem der beiden vorhergehenden Ansprüche, **dadurch gekennzeichnet, dass** die Umsetzung bei einer Kreisgasfahrweise erfolgt.

20. Verfahren nach einem der vorhergehenden Ansprüche, **dadurch gekennzeichnet, dass** man den Alkohol, Aldehyd und/oder das Keton als wässrige Lösung einsetzt.

21. Verfahren nach einem der vorhergehenden Ansprüche, **dadurch gekennzeichnet, dass** man den Ammoniak, das primäre oder sekundäre Amin als wässrige Lösung einsetzt.

22. Verfahren nach einem der vorhergehenden Ansprüche zur Herstellung von Monoaminodiglykol (ADG) und Morpholin durch Umsetzung von Diethylenglykol (DEG) mit Ammoniak.

23. Verfahren nach einem der Ansprüche 1 bis 21 zur Herstellung von N-(C₁₋₄-alkyl)-morpholin durch Umsetzung von Diethylenglykol (DEG) mit Mono(C₁₋₄-alkyl)amin.

24. Verfahren nach einem der Ansprüche 1 bis 21 zur Herstellung von 2-(2-Di(C₁₋₄-alkyl)aminoethoxy)ethanol und/oder Bis(2-di(C₁₋₄-alkyl)aminoethyl)ether durch Umsetzung von Diethylenglykol (DEG) mit Di(C₁₋₄-alkyl)amin.

25. Verfahren nach einem der Ansprüche 1 bis 21 zur Herstellung von Monoethanolamin (MEOA) und/oder 1,2-Ethylendiamin (EDA) durch Umsetzung von Monoethylenglykol (MEG) mit Ammoniak.

26. Verfahren nach einem der Ansprüche 1 bis 21 zur Herstellung von 1,2-Ethylendiamin (EDA) durch Umsetzung von Monoethanolamin (MEOA) mit Ammoniak.

27. Verfahren nach einem der Ansprüche 1 bis 21 zur Herstellung eines Polyetheramins durch Umsetzung eines entsprechenden Polyetheralkohols mit Ammoniak.

28. Verfahren nach einem der Ansprüche 1 bis 21 zur Herstellung von Piperazin und/oder Diethylentriamin (DETA) durch Umsetzung von N-(2-Aminoethyl)-ethanolamin (AEEA) mit Ammoniak.

29. Verfahren nach einem der Ansprüche 1 bis 21 zur Herstellung von Polyisobutenamin (PIBA) durch Umsetzung von Polyisobutenaldehyd mit Ammoniak.

30. Katalysator, **dadurch gekennzeichnet, dass** die katalytisch aktive Masse des Katalysators vor dessen Reduktion mit Wasserstoff sauerstoffhaltige Verbindungen des Aluminiums, Kupfers, Nickels und Kobalts und im Bereich von 0,2 bis 5,0 Gew.-% sauerstoffhaltige Verbindungen des Zinns, berechnet als SnO, enthält.

31. Katalysator nach dem vorhergehenden Anspruch, gekennzeichnet wie in einem der Ansprüche 2 bis 12 definiert.

## Claims

1. A process for preparing an amine by reacting a primary or secondary alcohol, aldehyde and/or ketone with hydrogen and a nitrogen compound selected from the group of ammonia, primary and secondary amines, in the presence of a supported copper-, nickel- and cobalt-containing catalyst, wherein the catalytically active material of the catalyst, before the reduction thereof with hydrogen, comprises oxygen compounds of aluminum, of copper, of nickel and of cobalt, and in the range from 0.2 to 5.0% by weight of oxygen compounds of tin, calculated as SnO.

2. The process according to claim 1, wherein the catalytically active material of the catalyst, before the reduction thereof with hydrogen, comprises in the range from 0.4 to 4.0% by weight of oxygen compounds of tin, calculated as SnO.

3. The process according to claim 1, wherein the catalytically active material of the catalyst, before the reduction thereof with hydrogen, comprises in the range from 0.6 to 3.0% by weight of oxygen compounds of tin, calculated as SnO.

4. The process according to any of the preceding claims, wherein the catalytically active material of the catalyst, before the reduction thereof with hydrogen, comprises in the range from 5.0 to 35% by weight of oxygen compounds of cobalt, calculated as CoO.

5. The process according to any of the preceding claims, wherein the catalytically active material of the catalyst, before the reduction thereof with hydrogen, comprises in the range from 10 to 30% by weight of oxygen compounds of cobalt, calculated as CoO.

6. The process according to any of the preceding claims, wherein the catalytically active material of the catalyst, before the reduction thereof with hydrogen, comprises in the range from
15 to 80% by weight of oxygen compounds of aluminum, calculated as Al₂O₃,
1.0 to 20% by weight of oxygen compounds of copper, calculated as CuO, and
5.0 to 35% by weight of oxygen compounds of nickel, calculated as NiO.

7. The process according to any of claims 1 to 5, wherein the catalytically active material of the catalyst, before the reduction thereof with hydrogen, comprises in the range from
30 to 70% by weight of oxygen compounds of aluminum, calculated as Al₂O₃,
2.0 to 18% by weight of oxygen compounds of copper, calculated as CuO, and
10 to 30% by weight of oxygen compounds of nickel, calculated as NiO.

8. The process according to any of the preceding claims, wherein the molar ratio of nickel to copper in the catalyst is greater than 1.

9. The process according to any of the preceding claims, wherein the catalytically active material of the catalyst does not comprise any rhenium and/or ruthenium.

10. The process according to any of the preceding claims, wherein the catalytically active material of the catalyst does not comprise any iron and/or zinc.

11. The process according to any of the preceding claims, wherein the catalytically active material of the catalyst does not comprise any oxygen compounds of silicon and/or of zirconium.

12. The process according to any of the preceding claims, wherein the BET surface area of the catalyst (ISO 9277:1995) is in the range from 30 to 250 m²/g.

13. The process according to any of the preceding claims, wherein the reaction is performed at a temperature in the range from 80 to 350°C.

14. The process according to any of the preceding claims, wherein the reaction is performed in the liquid phase at an absolute pressure in the range from 5 to 30 MPa, or in the gas phase at an absolute pressure in the range from 0.1 to 40 MPa.

15. The process according to any of the preceding claims, wherein the amine component (nitrogen compound) is used in 0.90 to 100 times the molar amount based on the alcohol, aldehyde and/or ketone used.

16. The process according to any of the preceding claims, wherein the catalyst is arranged as a fixed bed in the reactor.

17. The process according to any of the preceding claims, which is performed continuously.

18. The process according to the preceding claim, wherein the reaction is effected in a tubular reactor.

19. The process according to either of the two preceding claims, wherein the reaction is effected in a cycle gas method.

20. The process according to any of the preceding claims, wherein the alcohol, aldehyde and/or the ketone is used as an aqueous solution.

21. The process according to any of the preceding claims, wherein the ammonia or the primary or secondary amine is used as an aqueous solution.

22. The process according to any of the preceding claims for preparing monoaminodiglycol (ADG) and morpholine by reacting diethylene glycol (DEG) with ammonia.

23. The process according to any of claims 1 to 21 for preparing N-(C₁₋₄-alkyl)morpholine by reacting diethylene glycol (DEG) with mono (C₁₋₄-alkyl) amine.

24. The process according to any of claims 1 to 21 for preparing 2-(2-di(C₁₋₄-alkyl)aminoethoxy)ethanol and/or bis(2-di(C₁₋₄-alkyl)aminoethyl) ether by reacting diethylene glycol (DEG) with di(C₁₋₄-alkyl)amine.

25. The process according to any of claims 1 to 21 for preparing monoethanolamine (MEOA) and/or 1,2-ethylenediamine (EDA) by reacting monoethylene glycol (MEG) with ammonia.

26. The process according to any of claims 1 to 21 for preparing 1,2-ethylenediamine (EDA) by reacting monoethanolamine (MEOA) with ammonia.

27. The process according to any of claims 1 to 21 for preparing a polyetheramine by reacting a corresponding polyether alcohol with ammonia.

28. The process according to any of claims 1 to 21 for preparing piperazine and/or diethylenetriamine (DETA) by reacting N-(2-aminoethyl)ethanolamine (AEEA) with ammonia.

29. The process according to any of claims 1 to 21 for preparing polyisobuteneamine (PIBA) by reacting polyisobutenealdehyde with ammonia.

30. A catalyst, wherein the catalytically active material of the catalyst, before the reduction thereof with hydrogen, comprises oxygen compounds of aluminum, of copper, of nickel and of cobalt, and in the range from 0.2 to 5.0% by weight of oxygen compounds of tin, calculated as SnO.

31. The catalyst according to the preceding claim, characterized as defined in any of claims 2 to 12.

## Revendications

1. Procédé pour la production d'une amine par mise en réaction d'un alcool, d'un aldéhyde et/ou d'une cétone primaire ou secondaire avec de l'hydrogène et un composé azoté, choisi dans le groupe constitué par l'ammoniac, des amines primaires et secondaires, en présence d'un catalyseur contenant du cuivre, du nickel et du cobalt, fixé sur support, **caractérisé en ce que** la masse catalytiquement active du catalyseur, avant sa réduction par l'hydrogène, contient des composés oxygénés de l'aluminium, du cuivre, du nickel et du cobalt et dans la plage de 0,2 à 5,0 % en poids des composés oxygénés de l'étain, calculé en tant que SnO.

2. Procédé selon la revendication 1, **caractérisé en ce que** la masse catalytiquement active du catalyseur avant sa réduction par l'hydrogène contient dans la plage de 0,4 à 4,0 % en poids des composés oxygénés de l'étain, calculé en tant que SnO.

3. Procédé selon la revendication 1, **caractérisé en ce que** la masse catalytiquement active du catalyseur avant sa réduction par l'hydrogène contient dans la plage de 0,6 à 3,0 % en poids des composés oxygénés de l'étain, calculé en tant que SnO.

4. Procédé selon l'une quelconque des revendications précédentes, **caractérisé en ce que** la masse catalytiquement active du catalyseur avant sa réduction par l'hydrogène contient dans la plage de 5,0 à 35 % en poids des composés oxygénés du cobalt, calculé en tant que CoO.

5. Procédé selon l'une quelconque des revendications précédentes, **caractérisé en ce que** la masse catalytiquement active du catalyseur avant sa réduction par l'hydrogène contient dans la plage de 10 à 30 % en poids des composés oxygénés du cobalt, calculé en tant que CoO.

6. Procédé selon l'une quelconque des revendications précédentes, **caractérisé en ce que** la masse catalytiquement active du catalyseur avant sa réduction par l'hydrogène contient dans la plage de
15 à 80 % en poids des composés oxygénés de l'aluminium, calculé en tant qu'Al₂O₃,
1,0 à 20 % en poids des composés oxygénés du cuivre, calculé en tant que CuO, et
5,0 à 35 % en poids des composés oxygénés du nickel, calculé en tant que NiO.

7. Procédé selon l'une quelconque des revendications 1 à 5, **caractérisé en ce que** la masse catalytiquement active du catalyseur avant sa réduction par l'hydrogène contient dans la plage de
30 à 70 % en poids des composés oxygénés de l'aluminium, calculé en tant qu'Al₂O₃,
2,0 à 18 % en poids des composés oxygénés du cuivre, calculé en tant que CuO, et
10 à 30 % en poids des composés oxygénés du nickel, calculé en tant que NiO.

8. Procédé selon l'une quelconque des revendications précédentes, **caractérisé en ce que** dans le catalyseur le rapport molaire du nickel au cuivre est supérieur à 1.

9. Procédé selon l'une quelconque des revendications précédentes, **caractérisé en ce que** la masse catalytiquement active du catalyseur ne contient pas de rhénium et/ou de ruthénium.

10. Procédé selon l'une quelconque des revendications précédentes, **caractérisé en ce que** la masse catalytiquement active du catalyseur ne contient pas de fer et/ou de zinc.

11. Procédé selon l'une quelconque des revendications précédentes, **caractérisé en ce que** la masse catalytiquement active du catalyseur ne contient pas de composés oxygénés du silicium et/ou du zirconium.

12. Procédé selon l'une quelconque des revendications précédentes, **caractérisé en ce que** la surface BET du catalyseur (ISO 9277:1995) se situe dans la plage de 30 à 250 m²/g.

13. Procédé selon l'une quelconque des revendications précédentes, **caractérisé en ce qu'**on effectue la réaction à une température dans la plage de 80 à 350 °C.

14. Procédé selon l'une quelconque des revendications précédentes, **caractérisé en ce qu'**on effectue la réaction dans la phase liquide sous une pression absolue dans la plage de 5 à 30 MPa ou dans la phase gazeuse sous une pression absolue dans la plage de 0,1 à 40 MPa.

15. Procédé selon l'une quelconque des revendications précédentes, **caractérisé en ce qu'**on utilise le composant amine (composé azoté) en la quantité 0,90 à 100 fois molaire par rapport à l'alcool, l'aldéhyde et/ou la cétone utilisé(e).

16. Procédé selon l'une quelconque des revendications précédentes, **caractérisé en ce que** dans le réacteur le catalyseur est disposé sous forme de lit fixe.

17. Procédé selon l'une quelconque des revendications précédentes, **caractérisé en ce qu'**il est effectué en continu.

18. Procédé selon l'une quelconque des revendications précédentes, **caractérisé en ce que** la réaction a lieu dans un réacteur tubulaire.

19. Procédé selon l'une quelconque des revendications précédentes, **caractérisé en ce que** la réaction a lieu dans un mode opératoire avec gaz en circuit.

20. Procédé selon l'une quelconque des revendications précédentes, **caractérisé en ce qu'**on utilise l'alcool, l'aldéhyde et/ou la cétone sous forme de solution aqueuse.

21. Procédé selon l'une quelconque des revendications précédentes, **caractérisé en ce qu'**on utilise l'ammoniac, l'amine primaire ou secondaire, sous forme de solution aqueuse.

22. Procédé selon l'une quelconque des revendications précédentes, pour la production de monoaminodiglycol (ADG) et de morpholine par mise en réaction de diéthylèneglycol (DEG) avec de l'ammoniac.

23. Procédé selon l'une quelconque des revendications 1 à 21, pour la production de N-(alkyl(C₁-C₄))morpholine par mise en réaction de diéthylèneglycol (DEG) avec une mono(alkyl(C₁-C₄))amine.

24. Procédé selon l'une quelconque des revendications 1 à 21, pour la production de 2-(2-di(alkyl(C₁-C₄))aminoéthoxy)éthanol et/ou de bis (2-di-(alkyl(C₁-C₄))aminoéthyl)éther par mise en réaction de diéthylèneglycol (DEG) avec une di(alkyl(C₁-C₄))amine.

25. Procédé selon l'une quelconque des revendications 1 à 21 pour la préparation de monoéthanolamine (MEOA) et/ou de 1,2-éthylènediamine (EDA) par mise en réaction de monoéthylèneglycol (MEG) avec de l'ammoniac.

26. Procédé selon l'une quelconque des revendications 1 à 21 pour la préparation de 1,2-éthylènediamine (EDA) par mise en réaction de monoéthanolamine (MEOA) avec de l'ammoniac.

27. Procédé selon l'une quelconque des revendications 1 à 21 pour la préparation d'une polyétheramine par mise en réaction d'un polyétheralcool correspondant avec de l'ammoniac.

28. Procédé selon l'une quelconque des revendications 1 à 21 pour la préparation de pipérazine et/ou de diéthylènetriamine (DETA) par mise en réaction de N-(2-aminoéthyl)éthanolamine (AEEA) avec de l'ammoniac.

29. Procédé selon l'une quelconque des revendications 1 à 21 pour la préparation de polyisobuténamine (PIBA) par mise en réaction de polyisobuténaldéhyde avec de l'ammoniac.

30. Catalyseur, **caractérisé en ce que** la masse catalytiquement active du catalyseur, avant sa réduction par l'hydrogène, contient des composés oxygénés de l'aluminium, du cuivre, du nickel et du cobalt et dans la plage de 0,2 à 5,0 % en poids des composés oxygénés de l'étain, calculé en tant que SnO.

31. Catalyseur selon la revendication précédente, caractérisé comme défini dans l'une quelconque des revendications 2 à 12.
